(19) Europäisches Patentamt / European Patent Office / Office européen des brevets

(11) **EP 2 499 229 B1**

(12) **FASCICULE DE BREVET EUROPEEN**

(45) Date de publication et mention
de la délivrance du brevet:
**04.03.2015 Bulletin 2015/10**

(21) Numéro de dépôt: **10792979.6**

(22) Date de dépôt: **08.11.2010**

(51) Int Cl.:
***C12M 1/00*** (2006.01)

(86) Numéro de dépôt international:
**PCT/FR2010/052392**

(87) Numéro de publication internationale:
**WO 2011/058267 (19.05.2011 Gazette 2011/20)**

(54) **ENVELOPPE DE REACTION POUR UN REACTEUR PHOTOSYNTHETIQUE ET REACTEUR PHOTOSYNTHETIQUE ASSOCIE**

REAKTIONSGEHÄUSE FÜR EINEN FOTOSYNTHETISCHEN REAKTOR UND ZUGEHÖRIGER FOTOSYNTHETISCHER REAKTOR

REACTION CASING FOR A PHOTOSYNTHETIC REACTOR AND ASSOCIATED PHOTOSYNTHETIC REACTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.11.2009 FR 0957933**
**10.11.2009 US 259704 P**

(43) Date de publication de la demande:
**19.09.2012 Bulletin 2012/38**

(73) Titulaire: **Microphyt**
**34670 Baillargues (FR)**

(72) Inventeurs:
• **MULLER-FEUGA, Arnaud**
**F-34670 Baillargues (FR)**
• **LEMAR, Michel**
**F-69360 Saint Symphorien d'Ozon (FR)**

(74) Mandataire: **Chevalier, Renaud Philippe**
**Cabinet Germain & Maureau**
**BP 6153**
**69466 Lyon Cedex 06 (FR)**

(56) Documents cités:
WO-A2-2008/134010     WO-A2-2009/090549
FR-A1- 2 324 224     FR-A1- 2 621 323

**Description**

La présente invention se rapporte à une enveloppe de réaction pour un réacteur photosynthétique adapté pour la culture de microorganismes photosynthétiques, notamment d'algues, à un procédé de fabrication d'une telle enveloppe de réaction, à un réacteur photosynthétique associé et également à un procédé de culture de microorganismes photosynthétiques utilisant un tel réacteur.

Elle se rapporte plus particulièrement à une enveloppe de réaction conçue pour, d'une part, flotter sur une étendue d'eau et, d'autre part, délimiter un parcours d'écoulement diphasique gaz / milieu de culture liquide entre une première et une seconde ouverture de ladite enveloppe de réaction

La présente invention s'applique à la culture de tout organisme photosynthétique, c'est-à-dire de toute forme de vie susceptible de développement et de photosynthèse dans un milieu de culture nutritif approprié, en présence de rayonnement solaire et de gaz riche en carbone, tel que du dioxyde de carbone, les microalgues étant les principaux représentants de cette forme de vie.

L'analyse des performances comparées des organismes photosynthétiques conduit à privilégier la culture des microalgues qui sont les plus anciens acteurs de la photosynthèse sur notre planète. Contrairement aux plantes dressées, elles ne présentent aucun organe complexe et long à construire pour accéder à l'eau et à la lumière. Elles n'ont pas besoin de rigidifier leur tige au moyen de métabolites (cellulose, lignine) difficile à dégrader. Il s'ensuit une efficacité accrue des cultures de microalgues se traduisant par des productivités surfaciques pouvant atteindre 100 tonnes (poids sec) par hectare et par an contre 10 tonnes pour les meilleures plantes de plein champ. Toute la biomasse est utilisable alors que la récolte des grandes cultures ne concerne généralement que les graines, à l'exception notamment de la canne à sucre et des plantes fourragères qui laissent au moins en place le système racinaire.

Parmi les microorganismes photosynthétiques concernés par l'invention figurent plus particulièrement les végétaux aquatiques comme par exemple les microalgues, les protonémas de mousse, les petites macroalgues et les cellules isolées de plantes multicellulaires. Ces végétaux aquatiques présentent des propriétés intéressantes dans les domaines notamment de la pharmacie, de la nutrition humaine et animale, de la dermocosmétologie, de l'énergie et de l'environnement.

Comme pour la plupart des microorganismes photosynthétiques, l'accès à cette ressource consiste essentiellement dans la culture assistée dans des réacteurs adaptés. La lumière étant leur principal substrat, le milieu de culture doit présenter une interface optique recevant un flux de lumière. La difficulté de cultiver des microorganismes photosynthétiques tient au fait qu'ils constituent eux-mêmes des obstacles au passage de la lumière qui est leur principal substrat. La croissance de la culture va donc se stabiliser lorsque la lumière ne pénétrera plus dans l'épaisseur de la culture. Ce phénomène est appelé l'auto-ombrage.

La longueur du chemin optique, ou « light path length », permet de caractériser les différents modes de confinement, et se définit comme étant :

- la longueur du parcours de la lumière depuis son entrée dans la culture par une interface optique transparente jusqu'à une paroi opaque opposée ; ou
- la moitié de la distance qui sépare les deux interfaces optiques transparentes lorsque le confinement reçoit la lumière par deux interfaces optiques transparentes opposées.

Cette longueur du chemin optique varie entre quelques centimètres et quelques décimètres et détermine pour l'essentiel la production de biomasse par unité de temps et de surface optique (productivité surfacique en $g/m^2/j$) et la concentration de la culture (en g/L) en phase finale de croissance. Les différents modes de confinement qui sont mis en oeuvre pour assurer la culture des petits végétaux aquatiques peuvent ainsi être classés en fonction de cette longueur caractéristique.

La réaction de photosynthèse s'accompagne également d'une consommation de gaz carbonique ($CO_2$) et d'une production d'oxygène ($O_2$). L'excès d'oxygène inhibe la réaction, tandis que l'absence de gaz carbonique l'interrompt par défaut de substrat à transformer. Une interface gaz/liquide doit donc être aménagée pour les transferts de masse entre ces gaz et la phase liquide. Afin de favoriser ces échanges et d'éviter les hétérogénéités, la culture doit être le siège d'un mélange destiné à renouveler les organismes au niveau de l'interface optique susmentionnée et également au niveau de cette interface gaz/liquide.

Un premier mode de réalisation connu de réacteur photosynthétique consiste en un récipient ouvert de type bassin ou bac où la culture est maintenue par gravité et présente une surface libre réalisant à elle seule l'interface optique et l'interface liquide/gaz. La culture est mélangée à l'intérieur du bassin par un ou plusieurs dispositifs mécaniques de brassage, par exemple de type roue à aube. Les cultures en bassin ainsi réalisées peuvent couvrir des surfaces importantes et cette forme de réalisation est à l'origine de l'essentiel de la production mondiale actuelle de microalgues, qui atteint plusieurs milliers de tonnes de poids sec. Les organismes photosynthétiques produits par ce type de réacteur sont essentiellement :

- des algues dites extrêmophiles dont les milieux sont hostiles aux prédateurs et aux compétiteurs, comme par exemple les algues du type spiruline ou Dunaliella ; ou
- des algues dites dominantes qui supportent les sollicitations mécaniques ou les contaminations mieux que les autres, comme par exemple les algues du type Chlorella, Scenedesmus, Skeletonema, Odontella ou Nannochloropsis.

Un second mode de réalisation connu de réacteur photosynthétique consiste également en un récipient ouvert de type réservoir ou bac mais dont les dimensions sont inférieures à celles des bassins du premier mode de réalisation connu. Ces récipients présentent généralement des parois latérales transparentes au rayonnement solaire, de sorte que l'interface optique est constituée à la fois par la surface libre du milieu liquide et par les parois latérales transparentes.

Dans ce second mode de réalisation, il est classique de recourir à une injection d'air effectuée dans la partie basse du réservoir qui conduit à la formation de bulles d'air remontant dans le liquide jusqu'à la surface libre. La surface des bulles ainsi formées constitue l'interface gaz/liquide. En remontant vers la surface, les bulles entraînent la culture vers le haut, créant ainsi des mouvements convectifs qui peuvent s'étendre à tout le volume. Du dioxyde de carbone ($CO_2$) est parfois ajouté à l'air injecté pour apporter un surcroît de carbone selon une fraction molaire prédéfinie de quelques pourcents.

De volume inférieur à celui des bassins du premier mode de réalisation, les réservoirs du second mode de réalisation connu sont adaptés aux cultures plus contrôlées, en particulier aux cultures de microalgues destinées à la nutrition des larves de mollusques ou des proies vivante de larves de poissons en aquaculture. Le nettoyage fréquent de ces réservoirs ainsi que des inoculations pures et massives permettent de limiter les contaminations à l'intérieur du réservoir. Au nombre de plusieurs dizaines d'espèces les microalgues ainsi cultivées présentent des besoins en température et en lumière relativement voisins qui rendent possible leur culture dans des locaux communs.

Ces deux modes de réalisation sous la forme d'un récipient ouvert offrent une longueur de chemin optique de un à plusieurs décimètres.

Un troisième mode de réalisation connu de réacteur photosynthétique consiste en un réacteur clos dit photobioréacteur et qui comprend une boucle fermée à l'intérieur de laquelle circule le milieu de culture liquide, ladite boucle fermée comprenant une canalisation de réaction pourvue de tronçons de réaction réalisés dans un matériau transparent au rayonnement lumineux (ou à la lumière), et une canalisation de fermeture assurant la liaison entre les deux extrémités opposées de la canalisation de réaction.

Les photobioréacteurs, décrits notamment dans les documents GB 2 118 572 A, ES 2 193 860 A1, GB 2 331 762 A, ES 2 150 389 A1, FR 2 685 344 A1 et FR 2 875 511 A3, offrent des longueurs de chemin optique sensiblement plus faibles, de l'ordre de un à plusieurs centimètres, par rapport aux modes de réalisation avec récipient ouvert, et ils permettent d'atteindre des concentrations en organismes photosynthétiques de plusieurs grammes par litre à l'abri des contaminations aériennes. La canalisation de réaction des photobioréacteurs consiste généralement en des plaques ou tubes transparents, en verre ou matière plastique, d'épaisseur ou de diamètre de l'ordre du centimètre, qui sont connectées bout à bout par des coudes pour former ensemble une canalisation en forme de serpentin.

La canalisation de fermeture comprend un tube vertical dit ascendant, dans lequel le milieu liquide remonte, et un tube vertical descendant dans lequel le milieu liquide descend sous l'effet de la gravité.

Le système d'injection de gaz généralement mis en oeuvre dans les photobioréacteurs consiste en un gazosiphon, autrement appelé « gas-lift » ou dispositif d'ascenseur à gaz, c'est-à-dire en une injection de gaz à la base du tube vertical ascendant de la canalisation de fermeture ; ladite injection de gaz servant à la fois à mettre en circulation ou déplacer le milieu réactionnel liquide et à réaliser les échanges gaz-liquide. Le gazosiphon comporte en partie haute un réservoir de charge ou volume élargi dans lequel les vitesses de circulation plus faibles permettent la séparation gaz-liquide, et le tube vertical descendant de la canalisation de fermeture débouche dans le fond du réservoir de charge pour alimenter en liquide la canalisation de réaction.

Les photobioréacteurs susmentionnés appliquent le principe selon lequel la réaction n'a lieu que dans la phase liquide, autrement dit ces photobioréacteurs cherchent à minimiser le volume de gaz injecté dans le réacteur pour ne pas diminuer d'autant le volume du milieu de culture liquide, dans un souci de ne pas faire diminuer la production. Ainsi, dans ces photobioréacteurs, l'extraction d'oxygène est réalisée par le moyen du tube ascendant vertical défini ci-dessus ; ledit tube ascendant vertical formant une colonne à bulles d'air débouchant dans le réservoir de charge recevant le milieu de culture liquide, et comportant une injection de gaz en partie basse, opportunément de l'air enrichi de CO2. Comme décrit ci-dessus, les deux fonctions de circulation et de transfert gazeux sont confondues au sein de cet unique dispositif, appelé gazosiphon, qui crée une circulation verticale ascendante par échange de quantité de mouvement entre la masse liquide et les bulles de gaz résultant de l'injection. L'oxygène photosynthétique en sursaturation dans le liquide passe dans la phase gazeuse par balayage à l'air, tandis que le $CO_2$ passe en solution. Ces fonctions de dégazage et de carbonatation sont indispensables et interviennent simultanément et de façon indissociable au niveau de cet unique dispositif dans lequel la culture doit passer selon une fréquence élevée pour éviter une augmentation délétère de la teneur en oxygène dissous.

Les gazosiphon présentent l'inconvénient de générer des bulles de gaz qui remontent dans le tube ascendant vertical de la canalisation de fermeture des photobioréacteurs. La demanderesse a en effet observé le rôle délétère de ces bulles pour la culture de microorganismes dans les photobioréacteurs :

- d'une part, les bulles sollicitent mécaniquement les microalgues et peuvent nuire à des microorganismes fragiles ; et
- d'autre part, les bulles captent par effet tensio-actif les molécules qui présentent des propriétés surfactantes, et notamment les molécules organiques, débris cellulaires et les produits d'excrétion des cellules vivantes. Ces substances, normalement dispersées dans le milieu en l'absence de bulles, sont ainsi rassemblées sous forme d'agrégats à la surface libre du réservoir de charge lorsque les bulles éclatent. Les bactéries et champignons qui ne pourraient se développer en raison de la forte dilution de ces molécules organiques trouvent alors des substrats concentrés favorables à leur développement.

L'un des buts de la présente invention est d'éviter, ou du moins limiter, la formation des bulles pour :

- contenir le développement bactérien et fongique, par exemple pour rester compatible avec les normes sanitaires classiquement imposées dans la culture de microorganismes ; et pour
- limiter les sollicitations mécaniques dans le milieu de culture liquide, et ainsi permettre la culture de certains microorganismes fragiles qui étaient jusqu'à là exclus d'une telle culture en réacteur.

Dans une réalisation alternative au gazosiphon, la désoxygènation du milieu de culture liquide circulant dans le photobioréacteur est obtenue en faisant chuter gravitationnellement le milieu liquide dans un récipient à niveau constant. Le milieu de culture liquide est ici mis en circulation par un moyen de pompage, notamment du type pompe centrifuge, disposé dans la canalisation de réaction conçu pour non seulement compenser les pertes de charge dans la canalisation mais aussi élever la culture de la hauteur de la chute.

Bien que moins générateur de bulles, ce dispositif avec pompe centrifuge est aussi mécaniquement dommageable pour les microorganismes que le gazosiphon. En effet, pour vaincre les pertes de charge, il y a génération à chaque passage au droit du moyen de pompage d'efforts mécaniques qui peuvent contrarier la croissance des microorganismes et provoquer des mortalités au sein de la culture. Les performances de production s'en trouvent alors altérées, parfois de façon rédhibitoire.

Par exemple, on a constaté qu'il n'est pas possible de cultiver certaines microalgues dites fragiles dans des photobioréacteurs comportant des pompes centrifuges pour faire circuler la culture. Ces microalgues fragiles semblent d'autant plus sensibles aux sollicitations mécaniques qu'elles forment des chaînes et/ou qu'elles présentent des appendices tels que soies, flagelles, et spicules. Certaines microalgues, comme par exemple les algues du type Haematococcus pluvialis, perdent leurs flagelles et s'enkystent en formant une paroi cellulaire épaisse et résistante. Par contre, d'autres microalgues, comme par exemple les algues du type Chlorella vulgaris ou Nannochloropsis oculata, ne présentent pas d'appendice et possèdent une paroi cellulaire épaisse, de sorte que celles-ci résistent au passage dans les moyens de pompage, et notamment dans les pompes centrifuges.

Il est cependant difficile d'identifier la nature des sollicitations mécaniques influençant la survie et la croissance des microorganismes. La plupart des auteurs s'accordent pour dire que les cisaillements et les accélérations ont le plus d'influence délétère. Les cisaillements créent des tensions pouvant altérer l'intégrité cellulaire avec déchirement de la paroi des microorganismes et épanchement du cytosol. Les accélérations altèrent la structure de la cellule par augmentation du champ gravitationnel.

Les cellules vivantes sont mal préparées à ces efforts, et peut-être plus encore les cellules aquatiques qui vivent en équilibre hydrostatique et qui n'ont pas développé de structure capable de vaincre un champ gravitationnel. De surcroît, les cellules aquatiques sont sensibles à des valeurs seuil et probablement aussi aux variations et à la durée d'exposition. Dans l'état actuel des connaissances, il est difficile de prédire les effets mécaniques des conditions hydrodynamiques imposées aux cellules.

L'un des objets de la présente invention est de réduire les effets mécaniques imposées aux microorganismes, notamment les effets du type cisaillement et accélération, afin d'étendre le nombre d'espèces cultivables à l'intérieur du réacteur à celles qui sont les plus sensibles à ces effets mécaniques dommageables, autrement dit d'offrir un réacteur permettant la culture des microorganismes fragiles, comme par exemple les microalgues fragiles citées ci-dessus.

En outre, la demanderesse a observé que le rendement en culture des photobioréacteurs équipés de gazosiphon ou de pompe centrifuge était limité du fait notamment de la formation de bulles. En effet, la demanderesse a établi que le rendement en culture dépend en partie des phénomènes impliqués dans le transfert gaz-liquide pour éviter les pertes et réduire ce poste de dépense important. La modélisation du transfert gaz-liquide du dioxyde de carbone destiné à la réaction et de l'oxygène qu'elle produit nécessite la détermination de la vitesse de transfert qui est fonction du coefficient de transfert surfacique.

Le coefficient de transfert surfacique est un paramètre clé qui traduit les performances d'un système d'échange gaz/liquide à l'état stable. Ce coefficient de transfert surfacique est égale au produit du coefficient volumique de transfert de matière vers le liquide « KL » ($m.s^{-1}$) et de l'aire interfaciale rapportée au volume « a » ($m^{-1}$), où :

$$a = (\alpha_G.S)/V$$

a : Aire interfaciale rapportée au volume (m$^{-1}$) ;
$\alpha_G$ : coefficient de rétention de phase ;
S : Surface de contact (m$^2$) ; et
V : Volume du réacteur (m$^3$).

Le coefficient de transfert surfacique dépend donc de la géométrie du système d'échange gaz/liquide mais aussi des propriétés physicochimiques du liquide et du gaz. Dans le cas d'un échange gaz/liquide au sein d'une colonne à bulles verticale, la surface d'échange dépend du nombre de bulles et de leur taille. La population de bulles générée par une injection de gaz dans un liquide dépend du débit d'injection, de la géométrie de l'injecteur, et de la différence de pression de part et d'autre de celui-ci.

La présente invention a notamment pour but de fournir un réacteur photosynthétique qui permette la culture de masse des microorganismes photosynthétiques, et son extension aux espèces les plus fragiles, avec un réacteur qui réponde aux problématiques suivantes :

- réduire voire éviter les sollicitations mécaniques liées en général à l'agitation et à la mise en circulation du milieu de culture et qui diminuent les performances de survie et de croissance des microorganismes photosynthétiques tels que les microalgues, et plus particulièrement les microalgues en chaînes pourvues d'appendices ;
- réduire voire éviter la production de bulles de petites dimensions susceptibles de favoriser l'agrégation des molécules organiques et le développement des microorganismes hétérotrophes à qui elles servent de substrat ;
- tout en réalisant le transfert photonique, pour délivrer le rayonnement solaire aux microorganismes photosynthétiques, le transfert de masse ou transfert gaz/liquide indispensable pour apporter le carbone et évacuer l'oxygène, et le transfert thermique, pour évacuer les calories apportées par le rayonnement et maintenir la culture à la bonne température ; et
- tout en maintenant des conditions mécaniques préservant l'intégrité des cellules et évitant les échanges avec le milieu environnant de nature à se prêter aux contaminations et aux disséminations.

Concernant les trois modes de réalisation décrits ci-dessus, leur plus grande limitation au développement des productions d'algues vient du fait qu'ils sont destinés aux surfaces émergées de la planète, lesquelles servent prioritairement à l'urbanisation et aux cultures agricoles à vocation alimentaire, et présentent une rareté allant en s'accroissant avec la démographie humaine. Ce manque de surface limite sévèrement le développement par ces moyens des cultures de microalgues, notamment à vocation énergétique, qui devront, pour jouer un rôle significatif, occuper des surfaces considérables.

Pour répondre à cette problématique, la production des cultures d'algues a été envisagée dans des régions désertiques, mais cette perspective se trouve contrariée par la faible disponibilité en eau pour la fabrication du milieu liquide réactionnel autant que pour son refroidissement par évaporation.

Or les étendues d'eau, comme par exemple les plans d'eau naturels et artificiels des continents et surtout les mers, couvrent les surfaces les plus importantes de la Terre et ne sont encore que faiblement mis en valeur pour leur exposition à la lumière. Ces étendues d'eau ou surfaces aquatiques sont naturellement le siège de productions végétales considérables qui constituent le premier niveau des chaînes trophiques aquatiques en partie exploitées par les pêcheries mondiales. La production primaire des océans estimée actuellement à environ 10$^{11}$ tonnes par an est la plus importante de la planète. Ces masses végétales sont consommées par les herbivores sitôt produites, ce qui fait qu'elles sont peu visibles, sporadiques, diluées et difficiles à séparer de la masse d'eau ambiante, et jamais pures. Ceci explique que cette ressource abondante ne soit pas exploitée directement pour l'homme.

L'un des buts poursuivi par l'invention est de proposer une enveloppe de réaction, pour un réacteur photosynthétique adapté pour la culture de microorganismes photosynthétiques, pouvant d'être déployée sur une étendue d'eau ou à la surface des plans d'eau et des mers. Pour cela, l'invention se propose d'utiliser ces étendues d'eau recevant le rayonnement solaire pour assurer, outre la ressource en eau, deux fonctions essentielles des photobioréacteurs, à savoir la sustentation horizontale de surface et la stabilité thermique.

Il est connu, de l'état de la technique, plusieurs types de réacteurs photosynthétiques déployés sur une étendue d'eau afin de répondre à cette problématique d'utilisation des étendues d'eau.

Il est ainsi connu, de la demande de brevet FR 2 621 323, de prévoir un photobioréacteur comportant une enveloppe de réaction réalisée sous la forme d'un premier ensemble de tubes, parallèles, en matière plastique souple, tel que du polyéthylène, connectés entre eux à leurs deux extrémités à l'aide de deux collecteurs respectivement amont et aval. Ce premier ensemble de tubes assure le confinement du milieu de culture liquide. Le photobioréacteur comprend en outre un second ensemble de tubes placé en-dessous du premier ensemble de tubes, où les tubes de ce second ensemble sont destinés à être gonflés à l'air comprimé pour former un support pneumatique flottant. Un tel photobioréacteur présente de nombreux inconvénients, dont les principaux sont : une enveloppe de réaction complexe et coûteuse avec une succession de tubes et de collecteurs amont et aval qui alourdissent l'enveloppe de réaction et une structure complexe destinée à assurer la flottaison de l'enveloppe de réaction sur l'étendue d'eau imposée notamment par la présence de ces collecteurs.

Les document FR 2 361 060 et FR 2324224 décrivent respectivement un réacteur photosynthétique comportant une enveloppe de réaction réalisée sous la forme d'une série de tubes transparents reliés entre eux pour délimiter un trajet d'écoulement continu, en forme de serpentin, pour le milieu de culture liquide. Ces tubes sont logés dans un bâti pour former une structure flottante comportant des récipients de flottaison. Un tel réacteur présente de nombreux inconvénients, dont les principaux sont : une enveloppe de réaction complexe et coûteuse avec une succession de tubes raccordés entre eux à leurs extrémités, ces tubes nécessitant une structure complexe destinée à assurer la flottaison de l'ensemble.

Le document WO 2009/051479 A2 décrit un photobioréacteur comportant une enveloppe de réaction réalisée sous la forme d'une série de tubes transparents reliés entre eux par des pièces de couplage pour délimiter un trajet d'écoulement continu, en forme de serpentin, pour le milieu de culture liquide. Pour assurer la flottaison de ces tubes, le photobioréacteur comprend des flotteurs attachés sur les tubes. Un tel photobioréacteur présente de nombreux inconvénients, dont les principaux sont : une enveloppe de réaction complexe et coûteuse avec une succession de tubes raccordés entre eux à leurs extrémités par des pièces de couplage, ces tubes nécessitant l'ajout de flotteurs destinés à assurer la flottaison de l'ensemble.

Le document WO 2008/134010 A2 décrit un photobioréacteur pourvu d'une enveloppe de réaction réalisée sous la forme d'un tube en matériau flexible et transparent réalisant le confinement des volumes liquide et gazeux, et de flotteurs disposés sur les côtés du tube pour assurer la flottaison de l'ensemble. Le déploiement du volume de confinement est obtenu au moyen de raidisseurs et d'entretoises et la circulation diphasique gaz/liquide se fait dans un seul sens. Dans ce photobioréacteur, le tube doit être relié à ses deux extrémités à d'autres installations assurant la mise en mouvement et la fermeture de la boucle du liquide réactionnel.

Le document WO 2009/090549 A2 décrit un photobioréacteur pourvu d'une enveloppe de réaction réalisée sous la forme d'une poche tubulaire en matériau flexible et transparent. Dans ce photobioréacteur, l'apport en gaz ($CO_2$) au milieu de culture liquide peut se faire par diffusion passive de gaz sur une large superficie du milieu liquide, par injection de bulles de gaz, notamment en partie basse de la poche de réaction, avec tous les inconvénients mentionnés ci-dessus relatifs à la production de bulles.

Les réacteurs décrits dans les documents FR 2 621 323, FR 2 361 060, FR 2 324 224, WO 2009/051479 A2, WO 2008/134010 A2 et WO 2009/090549 A2 susmentionnés, présentent en outre un inconvénient supplémentaire commun : le nettoyage de l'enveloppe de réaction, à l'intérieur et à l'extérieur, est particulièrement complexe, et nécessite un démontage au moins partiel de l'enveloppe, sachant que le développement de salissures ou de biofilms sur les parois interne ou externe de l'enveloppe de réaction nuit à la transparence de l'enveloppe réactionnelle et donc au rendement de production des organismes photosynthétiques.

Afin de répondre en tout ou partie aux inconvénients et problématiques soulevés ci-dessus, la présente invention propose à cet effet une enveloppe de réaction pour un réacteur photosynthétique adapté pour la culture de microorganismes photosynthétiques, notamment d'algues, ladite enveloppe de réaction étant conçue pour, d'une part, flotter sur une étendue d'eau et, d'autre part, délimiter un parcours d'écoulement diphasique gaz / milieu de culture liquide entre une première et une seconde ouverture de ladite enveloppe de réaction, ladite enveloppe de réaction étant remarquable en ce qu'elle comporte deux gaines, respectivement externe et interne, réalisées au moins partiellement dans un matériau transparent au rayonnement lumineux, la gaine interne s'étendant à l'intérieur de la gaine externe de sorte que lesdites gaines délimitent entre elles un espace inter-gaine en liaison fluidique avec la première ouverture de l'enveloppe de réaction, en ce que la gaine externe présente une extrémité proximale ouverte et une extrémité distale fermée, et en ce que la gaine interne présente une extrémité proximale ouverte en liaison fluidique avec la seconde ouverture de l'enveloppe de réaction et une extrémité distale pourvue d'au moins un orifice de communication entre l'intérieur de la gaine interne et l'espace inter-gaine.

Avec cette enveloppe, le parcours d'écoulement diphasique s'effectue, entre la première ouverture et la seconde ouverture, dans l'espace inter-gaine et à l'intérieur de la gaine interne via l'orifice de communication ménagée à l'extrémité distale de la gaine interne.

Ainsi, cette enveloppe permet la production de microorganismes photosynthétiques, notamment de microalgues, par culture monoclonale en conditions contrôlées, pouvant être déployée à la surface d'une étendue d'eau (plan d'eau ou mer). Cette enveloppe devrait ainsi contribuer à la mise en valeur de ces étendues d'eau qui présentent les surfaces les plus abondantes et les moins mises en valeur de la planète, pour la production des microorganismes photosynthétiques.

En effet, cette enveloppe se propose d'exploiter plusieurs caractéristiques intrinsèques des étendues d'eau, à savoir :

- l'inertie thermique liée à l'importante capacité calorifique de l'eau qui, par l'échange avec le milieu de culture au travers de la gaine externe, permet de maintenir la température à des niveaux proches de l'optimum des microorganismes photosynthétiques cultivés,

- la capacité de sustentation des corps présentant une densité plus faible que l'eau qui permet d'assurer le maintien hydrostatique du volume de culture en surface selon l'horizontalité naturelle des plans d'eau, évitant ainsi les écoulements gravitaires et la formation de bulles indésirables ; et

- la transparence des étendues d'eau, lorsqu'ils ne reçoivent pas d'apport limoneux.

L'étendue d'eau peut servir de source d'eau locale pour la culture, mais il est souhaitable de la traiter afin d'en retirer les microorganismes indésirables, ainsi que certaines substances comme un excès des minéraux.

La présente enveloppe s'applique à la culture de tout organisme photosynthétique, c'est-à-dire de toute forme de vie susceptible de développement par le moyen de la photosynthèse dans un milieu de culture nutritif approprié, en présence de rayonnement solaire et de carbone, notamment sous forme de dioxyde de carbone.

Cette enveloppe de réaction, adapté aux étendues d'eau, permet de réaliser les fonctions suivantes :

- assurer dans la durée le confinement de la culture en empêchant les échanges de matière avec le milieu environnant qui se prêtent aux contaminations et aux disséminations, et pour cela résister aux sollicitations mécaniques notamment par les courants, les vents, et l'agitation de surface ;
- assurer le transfert photonique pour délivrer le rayonnement solaire aux microorganismes en culture ;
- assurer le transfert de masse gaz/liquide indispensable pour apporter le carbone et évacuer l'oxygène de la réaction ;
- assurer le transfert thermique, pour évacuer les calories apportées par le rayonnement et maintenir la culture à la bonne température ;
- se prêter à une diminution des coûts de revient de la biomasse produite par un coût modéré des moyens mis en oeuvre dans cette enveloppe.

L'enveloppe conforme à l'invention permet d'obtenir ces résultats, et présente pour cela un confinement particulier, avec deux gaines l'une dans l'autre délimitant un trajet d'écoulement continu des milieux réactionnels liquides et gazeux, qui se prête aux accroissements d'échelle vers de grandes surfaces; la production végétale des systèmes de production de microorganismes photosynthétiques étant, en effet, proportionnelle à cette surface selon un facteur appelé productivité surfacique, dont la valeur est de l'ordre de plusieurs dizaines de grammes de matière sèche par mètre carré et par jour.

De manière avantageuse, l'une au moins des deux gaines, respectivement externe et interne, est réalisée dans un matériau souple adapté pour permettre le pliage, le gonflage, la déformation transversale et/ou le fléchissement de ladite gaine. De préférence, les deux gaines sont réalisées dans un tel matériau souple.

Il est entendu par matériau souple un matériau qui se laisse déformer, plier, enrouler, fléchir sans se rompre ou se casser, tel qu'un matériau flexible ou ductile. Un tel matériau est particulièrement adapté pour l'enveloppe conforme à l'invention car il permet que :

- l'enveloppe dans son ensemble puisse être pliée ou enroulée afin d'être stockée sous une forme repliée ou enroulée, avant d'être déployée sur l'étendue d'eau par gonflage, un tel gonflage étant réalisé par remplissage au moyen du gaz et/ou du liquide avant d'établir leur circulation en aller et retour ;
- la gaine interne se déforme et fléchisse à l'intérieur de la gaine externe, de sorte que la paroi externe de la gaine interne frotte contre la paroi interne de la gaine externe nettoyant ainsi cette paroi interne de la gaine externe et cette paroi externe de la gaine interne ;
- la gaine externe se déforme et fléchisse, de sorte que la paroi interne de la gaine externe frotte contre la paroi externe de la gaine interne nettoyant ainsi cette paroi externe de la gaine interne et cette paroi interne de la gaine externe, et de sorte que la gaine externe frotte contre une gaine externe d'une enveloppe de réaction voisine ou adjacente nettoyant ainsi les parois externes de ces deux gaines externes ;
- les coûts de fabrication de ces enveloppes sont réduits avec l'emploi d'un matériau souple relativement économique.

Dans une réalisation particulière, l'enveloppe comprend en outre :

- une pièce externe de raccordement sur laquelle est montée hermétiquement l'extrémité proximale de la gaine externe, et sur laquelle est ménagée la première ouverture de l'enveloppe de réaction en liaison fluidique avec l'espace inter-gaine ; et
- une pièce interne de raccordement sur laquelle est montée hermétiquement l'extrémité proximale de la gaine interne, et sur laquelle est ménagée la seconde ouverture de l'enveloppe de réaction en liaison fluidique avec l'extrémité proximale de la gaine interne.

Ces pièces de raccordement, se présentant par exemple sous la forme de platines, sont particulièrement avantageuses pour réaliser le raccordement hermétique ou étanche des deux gaines avec une canalisation de fermeture assurant la liaison fluidique externe à l'enveloppe entre la première et la seconde ouverture de l'enveloppe de réaction. En outre, ces pièces de raccordement permettent de reprendre les efforts de tension longitudinale transmis par les gaines, et surtout par la gaine externe, pour les transmettre à une embarcation supportant la canalisation de fermeture dans laquelle est prévu un moyen de mise en circulation du milieu liquide.

Selon une caractéristique, l'extrémité proximale de la gaine interne est montée rotative sur la pièce interne de raccordement, de sorte que la gaine interne est libre de tourner et d'osciller à l'intérieur de la gaine externe, favorisant ainsi le nettoyage des parois.

Selon une autre caractéristique, la pièce interne de raccordement est montée à l'intérieur de la pièce externe de raccordement, limitant ainsi l'encombrement des pièces de raccordement avec les extrémités proximales des gaines situées sensiblement dans le même plan défini par les deux pièces.

De façon avantageuse, la pièce externe de raccordement comporte des moyens de couplage avec des moyens d'entraînement en rotation de ladite pièce externe de raccordement afin d'entraîner en rotation la gaine externe. Cet entraînement en rotation de la gaine externe est particulièrement avantageux pour réaliser le nettoyage de la paroi externe de la gaine externe.

Préférentiellement, la pièce interne de raccordement est libre en rotation dans la pièce externe de raccordement, afin que la rotation la gaine externe assure la rotation de la gaine interne par frottement entre les deux gaines. La rotation de la gaine externe se communique ainsi à la gaine interne, favorisant les mouvements des gaines et donc le nettoyage de leurs parois.

Dans un mode de réalisation particulier, la gaine interne, plus courte que la gaine externe, s'étend sur au moins 90 % de la longueur de la gaine externe, préférentiellement sur toute la longueur de la gaine externe diminuée de son diamètre, afin d'optimiser la longueur de l'écoulement diphasique gaz/liquide et donc les échanges entre les deux phases.

Selon une possibilité de l'invention, l'orifice de communication, prévu sur l'extrémité distale de la gaine interne, présente une zone de convergence, afin de réaliser une perte de charge dans l'écoulement diphasique gaz / milieu de culture liquide, et donc créer une surpression qui assure le gonflement de la gaine interne sur toute sa longueur.

Cette zone de convergence est par exemple réalisée sous la forme d'une réduction du diamètre de la gaine interne à l'extrémité distale ouverte de celle-ci.

Selon une autre possibilité de l'invention, l'enveloppe comprend en outre une troisième gaine en matériau souple s'étendant à l'intérieur de la gaine interne afin de permettre une injection ou une aspiration de gaz aux extrémités distales des deux gaines, respectivement externe et interne.

Dans le cas d'une injection de gaz cette troisième gaine, le retour de gaz se fait par l'espace inter-gaine, tandis que dans le cas d'une aspiration de gaz dans cette troisième gaine, l'aller du gaz se fait par la gaine interne et/ou l'espace inter-gaine. Cette dernière configuration est particulièrement adaptée aux plans d'eau agités où l'ondulation de surface tendra à déplacer le gaz injecté vers l'extrémité distale des deux gaines, respectivement externe et interne.

Dans un mode de réalisation particulier, l'enveloppe comprend en outre :

- des moyens de pincement ou de ligature amovibles conçus pour pincer ou ligaturer les deux gaines sur une zone intermédiaire située entre les extrémités proximales et distales respectives des deux gaines ;
- au moins un orifice intermédiaire de communication entre l'intérieur de la gaine interne et l'espace inter-gaine, ledit orifice intermédiaire de communication étant prévu sur la gaine interne entre son extrémité proximale et ladite zone intermédiaire de pincement ou de ligature ; et
- des moyens de fermeture dudit orifice intermédiaire de communication, notamment du type trappe, lesdits moyens de fermeture étant mobile entre une position ouverte et une position fermée.

Dans ce mode de réalisation particulier, il est possible de n'exploiter qu'un sous-volume de réaction, correspondant à la portion de l'enveloppe entre les extrémités proximales des gaines et la zone intermédiaire de pincement ou de ligature, afin d'inoculer et mettre en culture ce sous-volume, avant de mettre en culture le volume complet par retrait des moyens de pincement ou de ligature et fermeture des moyens de fermeture de l'orifice intermédiaire de communication.

Dans une configuration avantageuse, la section transversale de passage de la gaine interne est sensiblement identique à la section transversale de passage de l'espace inter-gaine.

Avec cette configuration, on permet que la vitesse de circulation du liquide dans la gaine interne est sensiblement équivalente à la vitesse de circulation dans l'espace inter-gaine, ce qui a l'avantage d'éviter l'apparition de zones de décantation et d'autres zones d'accélération susceptibles de créer des hétérogénéités non souhaitables le long du parcours.

Avantageusement, l'enveloppe comprend en outre une bande d'ombrage longitudinale recouvrant partiellement la gaine externe et réalisée dans un matériau opaque ou présentant une forte absorbance de la lumière, la bande d'ombrage étant notamment rapportée et positionnée temporairement sur la gaine externe ou fixée solidairement sur la gaine externe.

L'emploi d'une telle bande d'ombrage est particulièrement avantageux pour assurer une protection temporaire contre la luminosité de certaines cultures qui ne doivent pas être exposées à la pleine lumière tant que leur concentration est insuffisante pour les protéger par auto-ombrage.

L'invention se rapporte également à un réacteur photosynthétique adapté pour la culture de microorganismes photosynthétiques, notamment d'algues, comportant :

- au moins une enveloppe de réaction conforme à l'invention ;
- au moins une canalisation de fermeture assurant la liaison fluidique entre la première et la seconde ouvertures de ladite enveloppe de réaction ;
- au moins un moyen de mise en circulation disposé dans ladite canalisation de fermeture et conçu pour mettre en circulation le milieu de culture liquide dans la canalisation de fermeture et dans l'enveloppe de réaction ;
- au moins un moyen d'injection de liquide disposé dans ladite canalisation de fermeture et conçu pour permettre d'injecter du liquide dans l'enveloppe de réaction ;

- au moins un moyen d'injection de gaz disposé dans ladite canalisation de fermeture et conçu pour permettre d'injecter du gaz dans l'enveloppe de réaction ; et

- au moins un moyen d'échappement de gaz disposé dans ladite canalisation de fermeture et conçu pour permettre l'échappement du gaz injecté dans l'enveloppe de réaction.

Ce réacteur peut bien entendu comporter plusieurs enveloppes de réaction avec un moyen de mise en circulation commun à toutes ces enveloppes.

Avec un réacteur selon l'invention, le milieu de culture liquide et le gaz circulent simultanément au contact l'un de l'autre le long du parcours d'écoulement diphasique sensiblement horizontal car l'enveloppe de réaction, et donc les gaines délimitant ce parcours, flottent à la surface de l'eau qui est principalement horizontale (aux variations près induites par le vent, les vagues, les mouvements de surface, etc.), et échangent certains composants le long de leur parcours commun. Les échanges entre le milieu de culture liquide et le gaz sont proportionnels à la longueur des gaines, ce qui permet d'envisager de grands accroissements d'échelle.

Le réacteur et l'enveloppe de réaction conformes à l'invention sont ainsi spécialement conçus pour accroître l'efficacité du transfert gaz-liquide et pour diminuer les sollicitations mécaniques infligées aux organismes en culture afin d'étendre la production aux espèces fragiles.

En outre, le réacteur et l'enveloppe de réaction conformes à l'invention permettent de limiter la formation de bulles de petit diamètre et ainsi de réduire le développement des microorganismes hétérotrophes consommateurs d'oxygène. En effet, avec l'enveloppe de réaction selon l'invention, le transfert gaz/liquide ne se fait plus à l'intérieur d'une colonne à bulles verticale mais le long d'un parcours d'écoulement sensiblement horizontal dans lequel l'écoulement suit un régime du type diphasique horizontal, notamment du type écoulement stratifié ou écoulement à poches ou à bulles allongées.

Contrairement au principe mentionné ci-dessus selon lequel la réaction n'a lieu que dans la phase liquide, la demanderesse est partie du principe selon lequel le gaz fait partie intégrante de la réaction et doit être admis dans le volume réactionnel au même titre que le liquide. En privilégiant des régimes d'écoulement diphasiques horizontaux (stratifié, à poches ou à bulles allongées), la surface d'échange entre le gaz et le liquide est étendue à la totalité du parcours dans l'enveloppe de réaction avec une production de bulles nettement moins abondantes que dans le cas de certains réacteurs de l'art antérieur, réduisant de ce fait l'effet délétère observé pour ces bulles.

En outre, dans le réacteur selon l'invention, la mise en circulation du milieu de culture liquide est assurée par un ou plusieurs moyens de mise en circulation générant des forces de cisaillement et centrifuges réduites. La fonction de mise en circulation est dissociée de celle d'échange gaz-liquide contrairement au cas des réacteurs avec gazosiphon.

L'invention concerne également un procédé de culture de microorganismes photosynthétiques, notamment d'algues, utilisant un réacteur conforme à l'invention et comprenant les étapes suivantes :

- injection d'un milieu de culture liquide dans l'enveloppe de réaction selon un débit contrôlé avec le moyen d'injection de liquide ;

- injection d'un gaz dans l'enveloppe de réaction selon un débit contrôlé avec le moyen d'injection de gaz ;

- mise en circulation du milieu de culture liquide avec le moyen de mise en circulation ;

- contrôle du moyen de mise en circulation et du moyen d'injection de gaz pour établir dans l'enveloppe de réaction un régime d'écoulement diphasique gaz / milieu de culture liquide du type écoulement stratifié ou écoulement à poches ou à bulles allongées.

Selon une caractéristique, l'étape de contrôle comprend une étape de contrôle de la vitesse de circulation du liquide dans l'enveloppe de réaction entre environ 0,1 et 1,0 m/s, de préférence entre 0,1 et 0,5 m/s.

Selon une caractéristique, l'étape de contrôle comprend une étape de contrôle de la vitesse de circulation du liquide dans l'enveloppe de réaction de sorte que la vitesse de circulation dans la gaine interne est sensiblement équivalente à la vitesse de circulation dans l'espace inter-gaine ; ces vitesses de circulation pouvant chacune être comprise entre environ 0,1 et 1,0 m/s, de préférence entre 0,1 et 0,5 m/s.

Cette caractéristique est avantageuse pour éviter que n'apparaissent des zones de décantation et d'autres zones d'accélération susceptibles de créer des hétérogénéités non souhaitables le long du parcours. Ce contrôle est obtenu de préférence en ayant une configuration telle que la section transversale de passage de la gaine interne est sensiblement identique à la section transversale de passage de l'espace inter-gaine.

Selon une autre caractéristique, le moyen de mise en circulation comprend une hélice entraînée en rotation par un moteur, et la vitesse de rotation de l'hélice est inférieure à environ 1000 tours par minute, préférentiellement inférieure à environ 100 tours par minute.

Selon une caractéristique, l'injection du milieu de culture liquide et de gaz dans l'enveloppe de réaction est effectuée pour gonfler et déployer l'enveloppe sur la surface de l'étendue d'eau.

L'invention se rapporte aussi à un procédé de fabrication d'une enveloppe de réaction conforme à l'invention, comprenant les étapes suivantes :

- confection de la gaine interne, notamment par un procédé d'extrusion d'une matière plastique et de gonflage du plastique extrudé ;
- confection d'une nappe externe en matière plastique, notamment par un procédé de calandrage ;
- enveloppement de la gaine interne par la nappe externe jusqu'à une jonction de deux bords opposés ; et
- soudure de la nappe externe le long de ses deux bords opposés joints lors de l'étape d'enveloppement, de manière à former la gaine externe entourant la gaine interne.

Ces étapes peuvent être complétées d'une étape d'enroulement ou de pliage des gaines interne et externe ainsi réalisées.

Le procédé de fabrication conforme à l'invention est particulièrement économique et rapide.

D'autres caractéristiques et avantages de la présente invention apparaîtront à la lecture de la description détaillée ci-après, de plusieurs exemples de mise en oeuvre non limitatifs, faite en référence aux figures annexées dans lesquelles :

- la figure 1 est une vue schématique en coupe transversale d'une enveloppe conforme à l'invention ;
- la figure 2a est une vue schématique de dos d'une enveloppe conforme à l'invention, illustrant une pièce de raccordement commune aux gaines de l'enveloppe ;
- la figure 2b est une vue partielle en coupe longitudinale selon l'axe II-II de l'enveloppe illustrée sur la figure 2a, illustrant les extrémités proximales des gaines ;
- les figures 3a et 3b sont des vues schématiques en coupe longitudinale de deux enveloppes conformes à l'invention, illustrant deux variantes de réalisation d'un orifice de communication prévus à l'extrémité distale de la gaine interne ;
- la figure 4 est une vue schématique illustrant, de dessus et de profil, un réacteur conforme à l'invention comportant plusieurs enveloppes amarrées seulement d'un côté sur une embarcation flottante ;
- la figure 5 est une vue schématique illustrant de dessus un autre réacteur conforme à l'invention comportant plusieurs enveloppes amarrées des deux côtés sur respectivement une embarcation et un système de palonnier et d'émerillons ;
- la figures 6a est une vue partielle schématique de profil d'un réacteur conforme à l'invention comprenant une canalisation de fermeture intégrant des moyens de mise en circulation du milieu liquide selon un premier mode de réalisation ;
- la figure 6b est une vue en coupe transversale selon l'axe VI-VI de la partie droite du réacteur illustré sur la figure 6a ;
- la figures 7a est une vue partielle schématique de profil d'un réacteur conforme à l'invention comprenant une canalisation de fermeture intégrant des moyens de mise en circulation du milieu liquide selon un deuxième mode de réalisation ;
- la figure 7b est une vue en coupe transversale selon l'axe VII-VII de la partie droite du réacteur illustré sur la figure 7a ;
- la figure 7c est une vue en coupe transversale selon l'axe VII-VII de la partie gauche du réacteur illustré sur la figure 7a ;
- la figure 7d est une vue de dessus du réacteur illustré sur la figure 7a ;
- la figures 8a est une vue partielle schématique de profil d'un réacteur conforme à l'invention comprenant une canalisation de fermeture intégrant des moyens de mise en circulation du milieu liquide selon un troisième mode de réalisation ;
- la figure 8b est une vue de dos du réacteur illustré sur la figure 8a ;
- la figures 9a est une vue partielle schématique de profil d'un réacteur conforme à l'invention comprenant une canalisation de fermeture intégrant des moyens de mise en circulation du milieu liquide selon un quatrième mode de réalisation ;
- la figure 9b est une vue de dos du réacteur illustré sur la figure 9a ;
- les figures 10a et 10b sont des vues schématiques en coupe longitudinale d'une enveloppe conforme à l'invention, illustrant respectivement une étape de pincement de l'enveloppe afin de mettre en culture uniquement un sous-volume réactionnel de l'enveloppe et une étape de retrait des moyens de pincement afin de mettre en culture le volume complet de l'enveloppe;
- les figures 11a et 11b sont des vues schématiques de profil d'un réacteur conforme à l'invention comportant une enveloppe amarrée d'un côté sur une embarcation flottante, illustrant respectivement une étape de mise en place de l'enveloppe sur la canalisation de fermeture et une étape de déploiement de l'enveloppe sur l'étendue d'eau par gonflage ou remplissage ;
- la figure 12a est une vue schématique en coupe transversale de deux réacteurs conformes à l'invention et reliées entre eux via des canalisations de liaison ;
- la figure 12b est une vue schématique partielle de dessus des deux réacteurs illustrés en figure 12a ;
- la figure 13a est une vue schématique partielle de dessus d'un réacteur comportant plusieurs enveloppes de réaction raccordée à une canalisation de fermeture, intégrant un moyen de mise en circulation, commune à toutes les enveloppes ;
- la figure 13b est une vue en coupe longitudinale selon l'axe XIII-XIII du réacteur illustré sur la figure 13a ;
- la figure 14 est une vue schématique illustrant un système de fabrication d'une enveloppe conforme à l'invention ;
- la figure 15 est une vue schématique en perspective de réacteurs conformes à l'invention comportant des enveloppes amarrées sur une plateforme offshore.

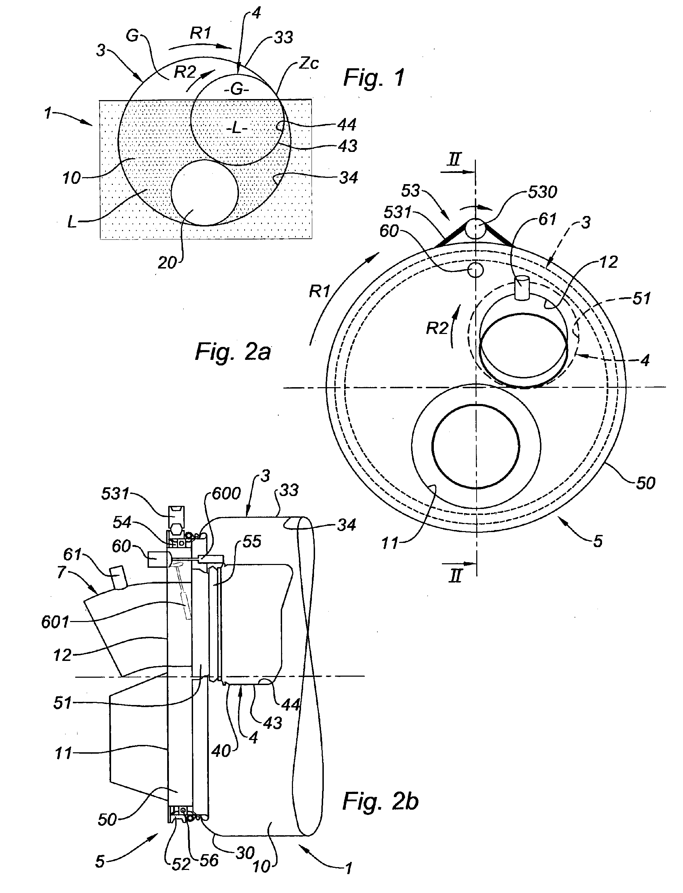

La description d'une enveloppe 1 de réaction conforme à l'invention pour un réacteur photosynthétique 2, ou photobioréacteur, est faite en référence aux figures 1 à 3 ; cette enveloppe 1 étant adaptée pour la culture de microorganismes photosynthétiques, notamment d'algues, et en particulier pour la culture de microorganismes photosynthétiques fragiles aux sollicitations mécaniques.

L'enveloppe 1 est conçue pour, d'une part, flotter sur une étendue d'eau et, d'autre part, délimiter un parcours d'écoulement diphasique gaz / milieu de culture liquide entre une première ouverture 11 et une seconde ouverture 12 de l'enveloppe 1. Cette enveloppe 1 comporte pour cela :

- une gaine externe 3 réalisée au moins partiellement dans un matériau souple et transparent au rayonnement lumineux, où la gaine externe 3 est allongée, avec notamment une forme générale tubulaire, et présente une extrémité proximale 30 ouverte et une extrémité distale 31 fermée, autrement dit se terminant en cul-de-sac ;
- une gaine interne 4 réalisée au moins partiellement dans un matériau souple et transparent au rayonnement lumineux, où la gaine externe 3 est allongée, avec notamment une forme générale tubulaire, s'étend à l'intérieur de la gaine externe 3 de sorte que ces deux gaines 3, 4 délimitent entre elles un espace inter-gaine 10 et présente une extrémité proximale 40 ouverte et une extrémité distale 41 pourvue d'au moins un orifice de communication 42 entre l'intérieur de la gaine interne 4 et l'espace inter-gaine 10 ;
- une pièce externe de raccordement 50 sur laquelle est montée hermétiquement l'extrémité proximale 30 de la gaine externe 3, et sur laquelle est ménagée la première ouverture 11 de l'enveloppe 1 qui est en liaison fluidique avec l'espace inter-gaine 10 ; et
- une pièce interne de raccordement 51 sur laquelle est montée hermétiquement l'extrémité proximale 40 de la gaine interne 4, et sur laquelle est ménagée la seconde ouverture 12 de l'enveloppe 1 qui est en liaison fluidique avec l'extrémité proximale 40 de la gaine interne 4.

La gaine externe 3 présente une longueur de plusieurs dizaines de mètres de long et un diamètre dit externe De décimétrique, par exemple compris entre environ 5 et 50 cm.

La gaine interne 4 présente :

- une longueur sensiblement équivalente à celle de la gaine externe 3, par exemple une longueur supérieure à environ 90 % de la longueur de la gaine externe 3 et préférentiellement égale à la longueur de la gaine externe 3 diminuée de son diamètre ; et
- un diamètre dit interne Di sensiblement plus faible que celui de la gaine externe 3.

La gaine interne 4 est ainsi déployée dans la gaine externe 3 de façon libre et sur environ toute sa longueur.

La Demanderesse a observé qu'il est préférable que les vitesses de circulation dans la gaine interne 4 et dans l'espace inter-gaine 10 soient aussi proches que possible l'une de l'autre, voire identiques, afin d'éviter que n'apparaissent des zones de décantation et d'autres d'accélération qui créent des hétérogénéités non souhaitables le long du parcours.

Pour cela, il faut et il suffit que les sections transversales de passage de la gaine interne 4 et de l'espace inter-gaine 10 soient identiques, ce qui peut se traduire comme suit dans le cadre de gaines 3, 4 tubulaire :

$$\Pi(De^2 - Di^2) / 4 = \Pi \, Di^2 / 4 \; ,$$

et donc on obtient la relation R1 suivante : $De = 2^{1/2}.Di$.

Opportunément, les vitesses de circulation dans la gaine interne 4 et dans l'espace inter-gaine 10 sont comprises entre 0,1 et 0,5 m/s.

Les deux gaines 3, 4 sont réalisées dans un matériau souple, autrement dit dans un matériau adapté pour permettre le pliage, le gonflage, la déformation transversale et/ou le fléchissement des gaines 3, 4.

Concernant la résistance des gaines 3, 4, la membrane de la gaine externe 3 doit pouvoir résister à l'effort de traction lié au déplacement de la masse de l'étendue d'eau, celui de l'air étant négligeable. Cet effort est sensiblement équivalent à l'effort de trainée d'un bateau de même longueur sur surface mouillée pour une même vitesse relative. Cette approche du calcul des efforts de traction auxquels sont soumises les gaines 3, 4, et qui sont transmis à un support d'amarrage (embarcation flottante E décrite ultérieurement, quai ou berge) par l'intermédiaire des pièces de raccordement 50, 51, est toutefois minorante puisqu'elle néglige les à-coups. Une marge de sécurité doit être prise, sachant que seule la gaine extérieure 3 reprend ces efforts.

La Demanderesse a ainsi établi une liste de matières plastiques utilisables pour la confection des gaines 3, 4, comprenant notamment le polyéthylène, le polypropylène, les polyamides (Nylon, Rylsan), les polytétrafluoréthylènes (PTFE), soit sous forme de membrane, soit sous forme de fibres tissées, soit sous forme composites de tissus calandrés ou enduits. Cette liste n'est bien entendu pas limitative et peut notamment être complétée par de nouveaux matériaux transparents apparaissant sur le marché.

Les membranes ou films réalisés dans ces matières plastiques présentent des résistances à la tension de plusieurs dizaines de kilogrammes par mètre linéaire, ce qui de l'ordre d'une aussière ou d'un cordage employé en marine de faible diamètre. Avec de tels matériaux, la gaine externe 3 peut donc résister aux efforts de traction induits par le vent. Il est donc avantageux de veiller à ce que le support d'amarrage, tel que l'embarcation flottante, transmette aussi peu d'à-coups que possible à l'enveloppe 1 et à ses gaines 3, 4.

Comme illustré sur les figures 2a et 2b, la pièce interne de raccordement 51 est montée à l'intérieur de la pièce externe de raccordement 50, de sorte que ces deux pièces de raccordement 50, 51 forment ensemble une platine de raccordement 5 ou de fixation, destinée à permettre le raccordement des gaines 3, 4 avec une canalisation de fermeture 7 décrite ultérieurement.

La pièce externe de raccordement 50 comporte des moyens de couplage 52 avec des moyens d'entraînement 53 en rotation de la pièce externe de raccordement 50 afin d'entraîner en rotation la gaine externe 3. Ainsi, l'extrémité proximale 30 de la gaine externe 3 est associée de façon hermétique à une fixation rotative, à savoir la pièce externe de raccordement 50. La vitesse de rotation mise en oeuvre par les moyens d'entraînement 53 est de l'ordre de quelques tours par jour.

Dans le mode de réalisation illustré sur les figures 2a et 2b, les moyens de couplage 52 sont réalisés sous la forme d'une poulie ménagée sur la périphérie cylindrique de la pièce externe de raccordement 50, et les moyens d'entraînement 53 comprennent un moteur rotatif pourvu d'un arbre de sortie 530 tournant sur lequel est montée une courroie ou une chaîne 531 en engrènement sur la poulie 52.

La pièce interne de raccordement 51, incluse dans la platine de raccordement 5, est libre en rotation dans la pièce externe de raccordement 50, afin que la rotation de la gaine externe 3 par les moyens d'entraînement 53 assure la rotation de la gaine interne 4 par frottement entre les deux gaines 3, 4. Ainsi, l'extrémité proximale 40 de la gaine interne 4 est montée rotative sur la platine 5. Autrement dit, l'extrémité proximale 40 de la gaine interne 4 est associée de façon hermétique à une fixation rotative, à savoir la pièce interne de raccordement 51.

La platine de raccordement 5 est destinée à reprendre les efforts de tension longitudinale transmis par les gaines 3, 4, et surtout la gaine externe 3, pour les transmettre à l'embarcation E d'amarrage.

Cette platine de raccordement 5 est en partie immergée dans l'étendue d'eau et est animée d'un mouvement de rotation, uniforme ou alternatif, de sorte qu'elle assure :

- la mise en rotation, ou une oscillation, des gaines 3, 4 avec une amplitude angulaire supérieure ou égale à 360°, soit sur au moins un tour complet, cette rotation étant illustrée par la flèche R1 sur les figures 1 et 2a ;
- la rotation libre de la gaine interne 4 à l'intérieur de la gaine externe 3, cette rotation étant illustrée par la flèche R2 sur les figures 1 et 2a ; et

- le maintien de l'intégrité / étanchéité du confinement réalisé par l'enveloppe 1, en permettant ces mouvements de rotation de façon hermétique.

Pour réaliser cette fixation hermétique et donc pour éviter les fuites, un joints 54 équipé d'un dispositif antifriction et d'étanchéité est prévu entre la fixation de la platine de raccordement 5 et la gaine externe 3 ; le volume interne de l'enveloppe 1 étant mis en pression, les fuites éventuelles vont vers l'extérieur, ce qui limite les risques de contamination. Un joints 55 équipé d'un dispositif antifriction et d'étanchéité est également prévu entre la fixation de la platine de raccordement 5 et la gaine interne 4, bien que les conséquences d'une fuite éventuelle à ce niveau soit sans gravité.

Pour diminuer l'effort d'entrainement en rotation de la platine de raccordement 5, la platine 5 est munie de roulements à billes 56 en-dessous de la poulie 52.

La rotation de la gaine externe 3 se communique à la gaine interne 4, dont la pièce de raccordement interne 51 est libre en rotation ainsi que décrit ci-dessus dans la platine 5, par leur frottement.

Comme visible en figure 2a et 2b, la fixation de la gaine interne 4 sur la platine 5 est légèrement décalée vers le haut pour :

- permettre une implantation de la sortie des gaz, autrement appelée moyen d'échappement de gaz 60, en partie haute ; et aussi pour
- favoriser le contact entre les deux gaines 3, 4, ce contact assurant l'entrainement en rotation, ou en oscillation, de la gaine interne 4 par la gaine externe 3.

Opportunément et comme visible en figure 2b, l'orifice du moyen d'échappement de gaz 60, c'est-à-dire de la sortie des gaz, est équipé d'une fermeture à flotteur 600 destinée à empêcher le passage du liquide.

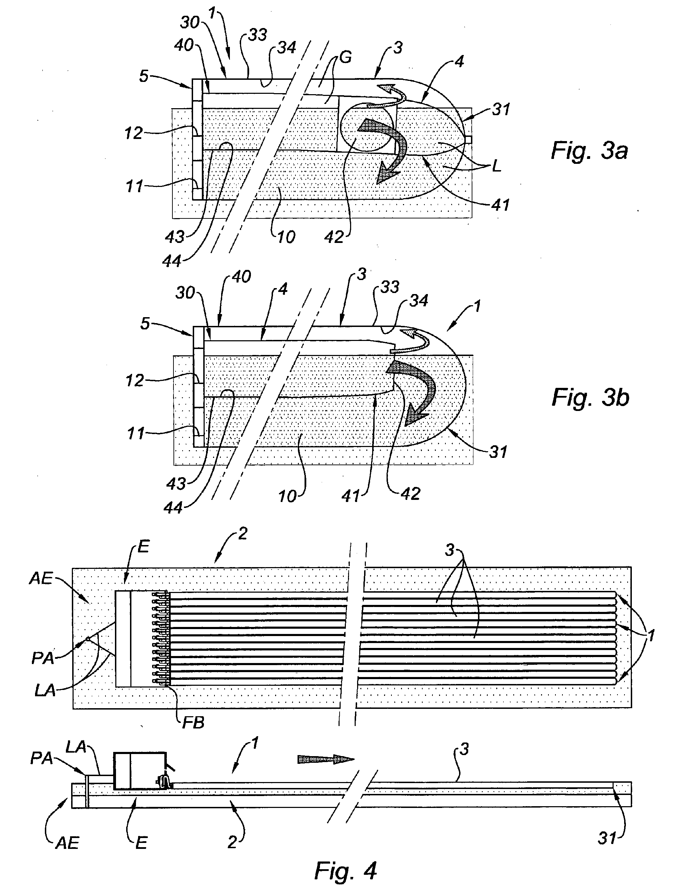

Les figures 3a et 3b illustrent deux enveloppes avec deux modes de réalisation distincts de l'orifice de communication 42. Dans le mode de réalisation illustré en figure 3a, l'orifice de communication 42 est ménagé sur la paroi latérale de la gaine interne 4, sous la forme d'une fenêtre éventuellement munie de renforts sur ses bords, où l'extrémité distale 41 de la gaine interne 4 est fixée solidairement, notamment par soudage ou par un pincement ou une ligature commune, à l'extrémité distale 31 de la gaine externe 3. Dans le mode de réalisation illustré en figure 3b, l'orifice de communication 42 est ménagé au bout de l'extrémité distale 41 de la gaine interne 4, de sorte que l'on parle d'une extrémité distale 41 ouverte et libre.

Le nettoyage des parois transparentes des gaines 3, 4 constitue l'un des avantages principaux de l'enveloppe 1 conforme à l'invention. En effet, ce nettoyage a pour but d'empêcher le développement de biofilms sur leurs parois respectives, afin d'éviter une diminution de la lumière transmise qui est préjudiciable puisqu'elle ralentit la réaction.

Pour réaliser ce nettoyage, plusieurs fonctions sont mises en oeuvre :

- les mouvements relatifs et le frottement des gaines externes 3 adjacentes les unes contre les autres permet le nettoyage de leurs parois externes 33 respectives, ces mouvements relatifs étant obtenus notamment par déformation et fléchissement des gaines externes 3 souples sous l'action de l'agitation de l'étendue d'eau induite notamment par le vent (clapot) ;
- la rotation de la gaine externe 3 sur au moins un tour (amplitude angulaire supérieure ou égale à 360°), permet d'étendre le nettoyage mentionné ci-dessus de leurs parois externes 33 à toute leur périphérie ;
- les mouvements relatifs entre les deux gaines 3, 4, provoquée par la rotation de la gaine externe 3 associée à l'action de l'agitation de l'étendue d'eau et à la souplesse de leurs matériaux, conduit au frottement de la gaine interne 4 contre la gaine externe 3 qui permet le nettoyage de la paroi interne 34 de la gaine externe 3 et le nettoyage de la paroi externes 43 de la gaine interne 4 ;
- la rotation de la gaine externe 3 sur au moins un tour (amplitude angulaire supérieure ou égale à 360°), permet d'étendre le nettoyage mentionné ci-dessus de la paroi interne 34 de la gaine externe 3 et le nettoyage de la paroi externes 43 de la gaine interne 4 à toute leur périphérie.

Ainsi, le contact entre les deux gaines 3, 4 d'une même enveloppe 1 a pour effet d'éviter le développement d'un biofilm et d'assurer le nettoyage à la fois de la paroi interne 34 de la gaine externe 3 et de la paroi externe 43 de la gaine interne 4. La rotation, ou une oscillation, d'amplitude angulaire supérieure ou égale à 360°, a pour objet de faire en sorte que ce nettoyage s'exerce sur toute la périphérie des gaines.

Comme illustré notamment sur les figures 1 et 3, le milieu de culture liquide L et le gaz G circulent simultanément au contact l'un de l'autre et échangent de la matière à travers une interface gaz-liquide le long du parcours de réaction allant de l'intérieur de la gaine interne 4 jusqu'à l'espace inter-gaine 10, ou inversement. La forme de cet espace inter-gaine 10 dépend notamment de la position relative des deux gaines 3, 4 et du régime d'écoulement, lui-même étant sous la dépendance des débits gazeux et liquide.

Comme illustré notamment sur les figures 1 et 3, la densité de la matière constitutive des gaines 3, 4 étant peu différente de celle de l'eau, les niveaux moyens de l'interface gaz-liquide dans l'espace inter-gaine 10 et dans la gaine interne 4 sont sensiblement identiques à celui du plan d'eau ; ces niveaux déterminant des lignes de flottaison intérieures et extérieures.

Comme visible en figure 1, la zone de contact ZC entre la gaine interne 4 et la gaine externe 3 est située au dessus de la ligne de flottaison. Il en résulte que l'espace inter-gaine 10 présente une section asymétrique en forme de croissant fermé, comme l'indique la figure 1. La gaine interne 4 tend à rester au contact de la gaine externe 3 par la force de capillarité exercée par le film liquide pris dans la zone de contact ZC entre les deux gaines 3, 4 dans l'espace inter-gaine 10. Le film liquide de la zone de contact ZC, dont l'épaisseur est faible, est entretenu par capillarité à partir du milieu de culture liquide L circulant dans l'espace inter-gaine 10.

La force de liaison ou de contact entre les deux gaines 3, 4 est faible et le contact peut être localement et temporairement défait par des mouvements du plan d'eau et les déformations des gaines 3, 4 qu'ils provoquent. La faiblesse du contact et les mouvements du plan d'eau font que le contact s'accompagne notamment de glissements et de frottements qui empêchent le développement d'un biofilm et favorisent le nettoyage des parois 43 et 34 impliquées dans le contact, à savoir la paroi interne 34 de la gaine externe 3 et le nettoyage de la paroi externes 43 de la gaine interne 4.

Comme décrit ci-dessus, la rotation de la gaine externe 3 est transmise à la gaine interne 4 par le frottement dans la zone de contact ZC, malgré les glissements qui altèrent la qualité de l'entrainement. Une certaine raideur de la gaine interne 4 est nécessaire pour éviter qu'elle ne se plisse et pour généraliser l'effet nettoyant aux parois 43 et 34. Pour éviter la consolidation du biofilm entre deux contacts, il suffit par exemple que la vitesse de rotation soit inférieure à la journée, ainsi que suggéré ci-dessus.

Les deux gaines 3, 4 canalisent le parcours des gaz et des liquides de la culture en un parcours d'écoulement diphasique gaz G / milieu de culture liquide L (ou parcours de réaction), formant un trajet aller et retour entre les ouvertures 11, 12 de l'enveloppe 1, dans la gaine interne 4 et dans l'espace inter-gaine 10 séparant la gaine interne 4 de la gaine externe 3.

La circulation de gaz dans les gaines 3, 4 crée une flottabilité positive répartie de façon homogène sur la longueur des gaines 3, 4 qui les maintient en surface. L'horizontalité est assurée naturellement par la sustentation de l'étendue d'eau et par la circulation du gaz. L'agitation de l'étendue d'eau sous l'effet du vent peut se traduire par des déformations longitudinales de la gaine externe 3, qui peuvent être transmises à la gaine interne 4, la flexibilité ou souplesse des gaines 3, 4 permettant le rappel ou retour aux formes initiales.

De préférence, il faut éviter que des plis n'apparaissent, avec une diminution sensible du volume réactionnel. En effet, de tels plis pourraient provoquer des à-coups et autres coups de bélier en cas d'agitation de l'étendue d'eau, ce qui occasionnerait des tensions brutales du matériau souple des gaines 3, 4 susceptibles de provoquer le déchirement des gaines 3, 4 en question. Pour cela, la gaine externe 3 doit être tendue en permanence grâce à une surpression de gonflement ; un contrôle de cette surpression devant permettre de maintenir la gaine externe à un niveau nominal compatible avec l'agitation de l'étendue d'eau et le bon fonctionnement de l'ensemble.

Ainsi, un procédé de culture de microorganismes photosynthétiques employant une telle enveloppe 1 comprend une étape de mise en pression de la gaine externe 3 consistant à créer une surpression de gonflement dans cette gaine externe 3.

La surpression de gonflement de la gaine externe 3 détermine, comme décrit ci-dessus, sa raideur, autrement dit sa résistance à la déformation liée à l'agitation du plan d'eau, et l'influence de cette dernière sur l'écoulement interne diphasique du gaz G et du liquide L ; cette surpression étant égale à la somme des pressions d'injection du gaz et du liquide dans le volume de la gaine externe 3.

Le contrôle de la surpression de gonflement a également pour objet de déceler des fuites. La sortie du volume gazeux en excédent se fait par le moyen d'échappement 60 décrit ci-dessus et pourvu d'un orifice aménagé en partie haute de la platine 5, ainsi qu'indiqué sur la figure 2b. Le gaz est par exemple canalisé jusqu'à un filtre qui permet d'éviter la rétro-contamination du réacteur avant d'être relâché à l'atmosphère ou recyclé. Un contrôle du débit de sortie du gaz peut être exercé, à l'aide d'un moyen de contrôle du débit gazeux 601, tel qu'une vanne à pointeau, afin de régler la hauteur du ciel (niveau du liquide ou de l'interface gaz/liquide) dans les gaines 3, 4.

Les figures 4 à 13 illustrent des réacteurs 2 photosynthétiques conformes à l'invention et adaptés pour la culture de microorganismes photosynthétiques, notamment d'algues. Chaque réacteur 2 comprend :

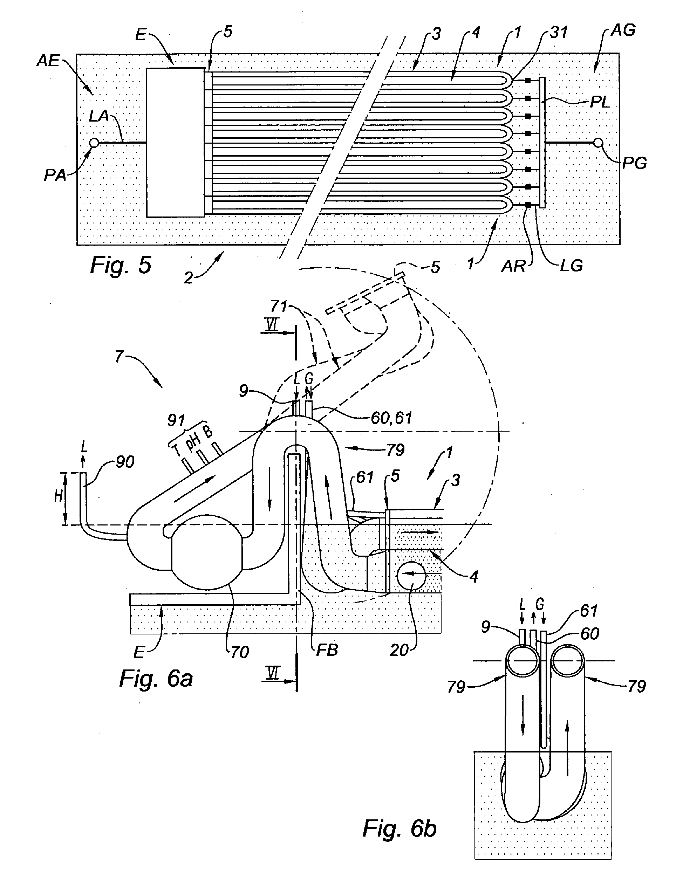

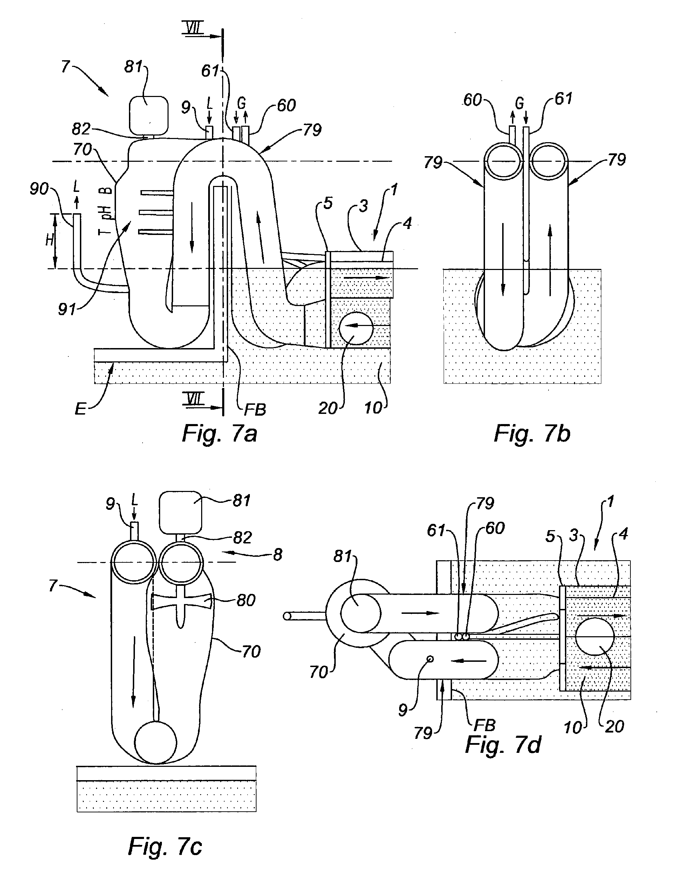

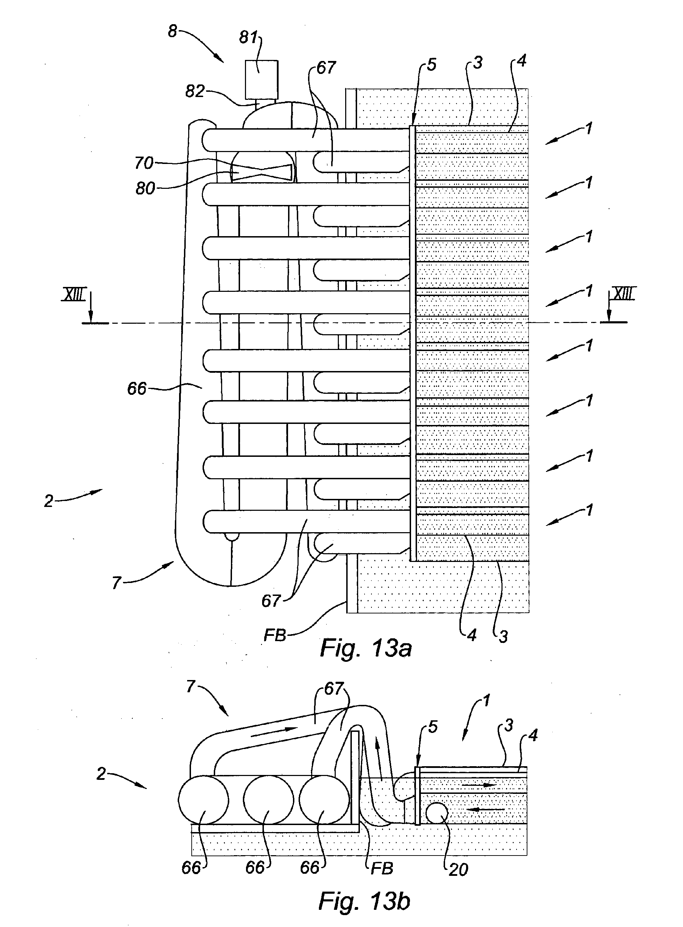

- au moins une enveloppe 1 conforme à l'invention ;
- au moins une canalisation de fermeture 7 (visible notamment sur les figures 6, 7, 8, 9, 12 et 13) assurant la liaison fluidique entre la première 11 et la seconde ouverture 12 de l'enveloppe 1, lesdits ouvertures 11, 12 étant ménagées dans la platine 5 de raccordement sur laquelle est raccordée de façon étanche ladite canalisation de fermeture 7 ;
- au moins un moyen de mise en circulation 8 (visible sur les figures 7c et 13a) disposé dans la canalisation de fermeture 7 et conçu pour mettre en circulation le milieu de culture liquide L dans la canalisation de fermeture 7 et dans l'enveloppe 1 ;
- au moins un moyen d'injection de liquide 9 disposé dans la canalisation de fermeture 7 et conçu pour permettre d'injecter du liquide L dans l'enveloppe 1 ;
- au moins un moyen d'injection de gaz 61 disposé dans la canalisation de fermeture 7 et conçu pour permettre d'injecter du gaz G dans l'enveloppe 1; et
- au moins un moyen d'échappement de gaz 60 disposé dans la canalisation de fermeture et conçu pour permettre l'échappement du gaz G injecté dans l'enveloppe 1.

Le réacteur 2 peut comprendre deux moyens d'injection de liquide 9 distincts permettant d'injecter respectivement le milieu de culture liquide et l'inoculum dans le réacteur 2. Ces moyens d'injection 9 se présentent sous la forme de ports d'injection permettant une connexion à une source avec contrôle de l'asepsie.

Le réacteur 2 peut comprendre :

- un ou plusieurs capteurs 91 disposés sur la canalisation de fermeture 7 et adaptés pour fournir les signaux nécessaires au contrôle de la réaction, notamment des signaux représentatifs des paramètres physiques, chimiques, ou biologiques de la qualité de la culture, tels que la température, le pH, le taux d'oxygène dissous et la turbidité du milieu liquide, etc. ces contrôles servant notamment à réguler les injections de gaz et de liquide dans le réacteur 2 ;
- des moyens de contrôle de la stérilité des milieux gazeux et liquides entrant et sortant de l'espace confiné par le réacteur 2 ;
- des boucles de régulation destinées à réguler les principaux apports nutritifs de la culture, notamment, l'admission de milieu stérile par la concentration en matière sèche, le pH par l'injection de $CO_2$.

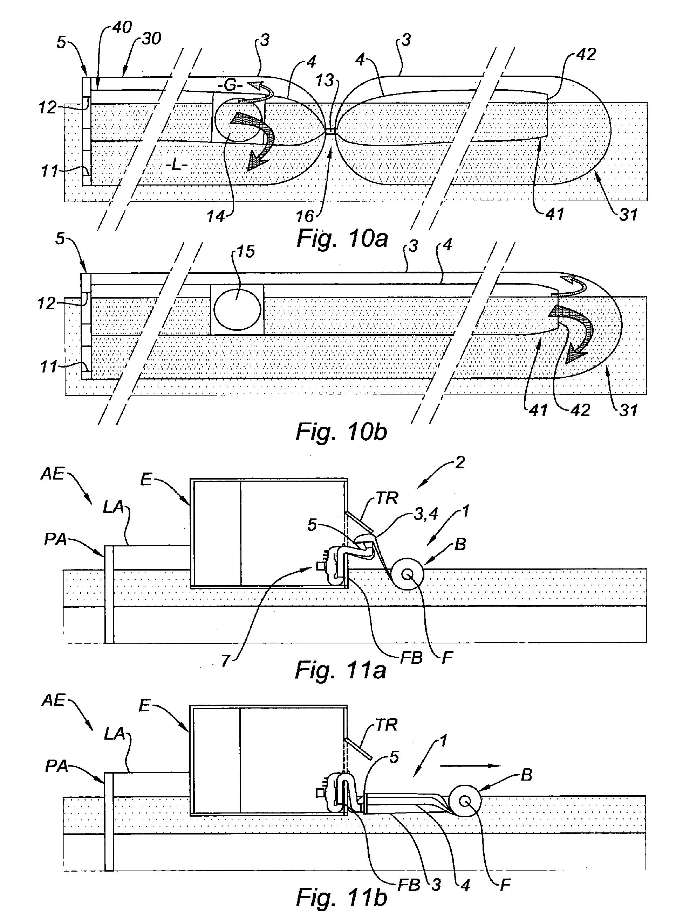

La canalisation de fermeture 7 assure la fermeture du parcours fluidique en boucle entre la première 11 et la seconde 12 ouvertures de l'enveloppe 1. La canalisation de fermeture 7 est réalisée dans une matière non transparente au rayonnement solaire et/ou peut être disposée à l'abri de la lumière à l'intérieur d'un local fermé ou d'une embarcation E fermée comme visible notamment sur les figures 4 et 11.

Le moyen de mise en circulation 8, interposé sur la ou les canalisations de retour 7, a pour fonction la mise en circulation du milieu de culture liquide dans l'enveloppe 1. De préférence, le moyen de mise en circulation 8 est choisi pour générer des forces de cisaillement et centrifuges réduites. Il est cependant possible d'utiliser tous types de moyens de pompage et notamment des pompes centrifuges sans que cela ne sorte du cadre de l'invention.

De préférence, la fonction de mise en circulation est dissociée de celle d'échange de masse gaz-liquide qui est assurée à travers leur interface dans les gaines 3, 4 de l'enveloppe 1 et qui s'exerce sur toute leur longueur. Cependant, une forme particulière non illustrée de réalisation du réacteur selon l'invention peut comporter la mise ne oeuvre d'une solution de type gazosiphon, décrite ci-dessus, qui assure à la fois le pompage et l'échange de masse gaz-liquide, sans que cela ne sorte du cadre de l'invention.

Le réacteur 2 est particulièrement avantageux en appliquant le principe selon lequel le gaz fait partie intégrante de la réaction et doit être admis dans le volume réactionnel au même titre que le liquide par la mise en oeuvre d'un écoulement diphasique horizontal dans les gaines 3, 4 de l'enveloppe 1 flottant sur l'étendue d'eau ;

ces deux gaines 3, 4 souples incluses l'une dans l'autre créant un parcours aller-retour du gaz et du liquide.

Avec une telle enveloppe 1, et donc avec un tel réacteur 2, quatre configurations possibles de parcours diphasique sont envisageables :

- un premier parcours : circulation à co-courant avec le liquide L et le gaz G entrant dans l'enveloppe 1 par la seconde ouverture 12 et sortant de l'enveloppe 1 par la première ouverture 11, de sorte que le liquide L et le gaz G effectuent un trajet aller dans la gaine interne 4 et un trajet retour dans l'espace inter-gaine 10 ;

- un second parcours : circulation à co-courant avec le liquide L et le gaz G entrant dans l'enveloppe 1 par la première ouverture 11 et sortant de l'enveloppe 1 par la seconde ouverture 12, de sorte que le liquide L et le gaz G effectuent un trajet aller dans l'espace inter-gaine 10 et un trajet retour dans la gaine interne 4 ;

- un troisième parcours : circulation à contre courant avec le liquide L entrant dans l'enveloppe 1 par la seconde ouverture 12 et sortant de l'enveloppe 1 par la première ouverture 11 et avec le gaz G entrant dans l'enveloppe 1 par la première ouverture 11 et sortant de l'enveloppe 1 par la seconde ouverture 12, de sorte que le liquide L effectue un trajet aller dans la gaine interne 4 et un trajet retour dans l'espace inter-gaine 10 et que le gaz G effectue un trajet aller dans l'espace inter-gaine 10 et un trajet retour dans la gaine interne 4 ; et

- un quatrième parcours : circulation à contre courant avec le liquide L entrant dans l'enveloppe 1 par la première ouverture 11 et sortant de l'enveloppe 1 par la seconde ouverture 12 et avec le gaz G entrant dans l'enveloppe 1 par la seconde ouverture 12 et sortant de l'enveloppe 1 par la première ouverture 11, de sorte que le liquide L effectue un trajet aller dans l'espace inter-gaine 10 et un trajet retour dans la gaine interne 4 et que le gaz G effectue un trajet aller dans la gaine interne 4 et un trajet retour dans l'espace inter-gaine 10.

Dans le cas du premier parcours, le gaz G et le milieu liquide de culture L sont injectés dans la gaine interne 4 au niveau de la seconde ouverture 12 ménagée dans la platine 5 et s'échappent de cette gaine interne 4 via l'orifice de communication 42 prévue à l'extrémité distale 41, puis reviennent au niveau de la platine 5 dans l'espace inter-gaine 10.

Dans le cas du quatrième parcours, le gaz G est injecté dans la gaine interne 4 tandis que le milieu liquide de culture L est injecté dans l'espace inter-gaine 10 ; le retour du gaz G se faisant dans l'espace inter-gaine 10 et celui du liquide L dans la gaine interne 4.

Dans tous les cas, le liquide L est repris au retour par au moins une pompe située dans la ou les canalisations de retour 7 pour être réintroduit dans le même parcours aller, tandis que le gaz G est soit libéré dans l'atmosphère soit recyclé via le moyen d'échappement de gaz 60.

De façon préférentielle, l'orifice de communication 42, prévu sur l'extrémité distale 41 de la gaine interne 4, présente une zone de convergence afin de réaliser une perte de charge dans l'écoulement diphasique gaz / milieu de culture liquide. Dans le mode de réalisation illustré en figure 3b, l'extrémité distale 41 ouverte de la gaine interne 4 présente un diamètre réduit pour créer cette perte de charge singulière ; la surpression créée par cette zone de convergence ou restriction assurant le gonflement de la gaine interne 4 sur toute sa longueur.

Éventuellement, une troisième gaine (non illustrée), également réalisée dans un matériau souple et transparent au rayonnement lumineux, de paroi suffisamment raide pour pouvoir subir une dépression sans collapsus et de petit diamètre (comparativement au diamètre de la gaine interne 4), est située à l'intérieur de la gaine interne 4 pour permettre une injection ou une aspiration de gaz à l'extrémité distale 31, 41 des deux gaines 3, 4.

Comme visible en figure 1, la circulation du gaz G et du liquide L, à contre-courant ou à co-courant, se fait le long d'un parcours horizontal sensiblement rectiligne où le gaz G se rassemble dans un volume situé dans la partie supérieure ou haute de la cavité délimitée par les deux gaines 3, 4.

Il se crée ainsi une interface entre le gaz G et le liquide L qui est le siège des transferts liés à la réaction de photosynthèse. La forme longitudinale de cette interface, et notamment son caractère continu ou discontinu, caractérise ce que l'on appelle le régime d'écoulement. Sans considérer l'influence de l'agitation du plan d'eau, les régimes d'écoulement dans la gaine interne 4 sont sensiblement les mêmes que dans une canalisation horizontale de section circulaire. Ces écoulements ont été décrits sous les noms d'écoulement stratifié ou à poches ou à bulles allongées. Les régimes d'écoulement dans l'espace inter-gaine 10 sont légèrement perturbés par la présence de la gaine interne 4, mais auront des caractéristiques sensiblement identiques à l'égard du transfert de masse.

Concernant les écoulements diphasiques dans des conduites horizontales, des travaux ont en effet mis en évidence plusieurs régimes d'écoulement selon les conditions de vitesse, de diamètre, de température, de nature, de pression des fluides circulant, à savoir notamment :

- écoulement à bulles dispersées, dispersed bubbles flow, de typologie de Mandhane AD ; et
- écoulement à bulles allongées, ou elongated bubbles flow, de typologie de Mandhane I ;
- écoulement stratifié, ou stratified flow, avec un écoulement stratifié ondulé et avec un écoulement stratifié lisse, de typologie de Mandhane SS et SW ;
- écoulement à poches, ou slug flow, de typologie de Mandhane I ;
- écoulement annulaire, ou annular mist flow, de typologie de Mandhane AD.

Dans le cas de la présente invention, les régimes d'écoulement privilégiés se situent donc au niveau de la transition SS/I dans la typologie de Mandhane, c'est-à-dire entre le régime stratifié et le régime à poches ou à bulles allongées. Dans le régime stratifié, l'interface gaz/liquide est constituée par la surface libre, dont la largeur varie avec le niveau du liquide dans les gaines 3, 4. Dans le régime à poches ou à bulles allongées, l'interface gaz/liquide est constituée par le plancher et le plafond de la poche ou de la bulle allongée.

Les transferts de masse étant proportionnels à la longueur du parcours, l'effet de celle-ci sur les performances de la réaction est réduit, ce qui permet d'envisager de grands accroissements d'échelle. Les gaines 3, 4 peuvent présenter des longueurs de plusieurs centaines de mètres.

Dans le cas particulier du premier parcours décrit ci-dessus, le gaz G et le liquide L sont injectés simultanément dans la gaine interne 4. Afin d'éviter le reflux du gaz G à contre courant du liquide L, le gaz G est injecté de préférence en un point d'injection 61 situé à l'aval d'un point bas du plafond de la canalisation de fermeture 7 qui fait office d'anti-retour, ainsi qu'illustré sur les figures 6, 7, 8 et 9. La séparation du liquide L et du gaz G est complétée par le positionnement de la première ouverture 11, formant la sortie de l'espace inter-gaine 10, en partie basse de la platine 5. Cette séparation est essentielle pour ne pas altérer les performances du moyen de mise en circulation 8.

La sortie du volume de liquide L en excédent dans le réacteur se fait notamment par un déversoir constitué par une canalisation de sortie 90 (visible sur les figures 6a, 7a, 8, 9 et 12) en communication avec la canalisation de fermeture 7. L'extrémité libre de cette canalisation de sortie 90 est callée à une hauteur H réglable par rapport au plan d'eau ; cette hauteur H, visible sur les figures 6a et 7a, déterminant la surpression de gonflement de la gaine externe 3 et pouvant être réglée pour stabiliser l'écoulement interne en fonction de l'agitation du plan d'eau. Cette hauteur H est de l'ordre de quelques centimètres et peut dépasser plusieurs décimètres pour des gaines 3, 4 de grande longueur.

Le point de communication de cette canalisation de sortie 90 du liquide L avec la canalisation de fermeture 7 est placé aussi loin que possible et de préférence à l'amont du ou des points d'injection 9 de milieu liquide L stérile, comme illustré sur les figures 6a et 7a, les flèches illustrant sur les figures le sens de circulation du milieu liquide L. C'est en effet à la sortie de cette canalisation de sortie 90 que se fait la récolte de la culture et il faut éviter les courts circuits qui la dilueraient.

Cette canalisation de sortie 90, formant déversoir, constitue une rupture du confinement du milieu de culture liquide L. Afin d'éviter les rétro-contaminations de la culture en cours, la canalisation de sortie 90 peut utilement avoir une longueur de plusieurs mètres et être maintenue stérile par des nettoyages périodiques.

Comme visible sur les figures 4, 5 et 11, la canalisation de fermeture 7, le moyen de mise en circulation 8 et la platine 5 sont fixés sur une embarcation E reposant sur l'eau ; cette embarcation E pouvant être du type flottant et former une embarcation ou barge flottante, ou pouvant être du type ponton avec des poutres ou madriers plantés dans le fond de l'étendue d'eau.

Cette embarcation E peut comprendre un espace fermé, ou espace technique, dans lequel sont disposés, à l'abri des intempéries, la canalisation de fermeture 7 et le moyen de mise en circulation 8, et une paroi ou franc-bord FB sur laquelle les gaines 3, 4 sont fixées à cette embarcation E par l'intermédiaire de la platine 5 ; les extrémités distales 31, 41 de ces gaines 3, 4 étant laissées libres, ce qui leur permet de s'aligner dans le sens du déplacement relatif de la masse d'eau qui supporte l'ensemble, et a pour effet de réduire les efforts de trainée liés au déplacement de cette masse d'eau support. Un tel ensemble productif optimise l'utilisation des moyens communs tels que l'embarcation E et les fonctionnalités embarquées.

Dans les modes de réalisation illustrés sur les figures 4 et 11, l'embarcation E est flottante et amarrée dans le fond de l'étendue d'eau, ici peu profonde, par un amarrage AE unique de l'embarcation E, de sorte que les gaines externes 3 des enveloppes 1 peuvent s'aligner dans le champ de courant induit par le vent à partir de cette embarcation E oscillant librement autour de son amarrage unique AE. Cet amarrage AE comprend un point d'amarrage PA, réalisé notamment sous la forme d'une poutre ou tige plantée verticalement dans le fond de l'étendue d'eau, et des liens d'amarrage LA reliant l'embarcation E au point d'amarrage PA et laissant ladite embarcation E libre en rotation autour du point d'amarrage PA. Lorsque l'amarrage AE se fait en un point unique PA, comme dans le cas des modes de réalisation illustrés sur les figures 4 et 11, il faut prévoir suffisamment d'espace pour que l'ensemble puisse éviter autour de ce point sous l'influence des courants, et notamment ceux induits par le vent.

Dans une autre forme de réalisation illustrée sur la figure 5, les extrémités distales 31 des gaines externes 3 sont amarrée dans le fond de l'étendue d'eau, ici peu profonde, par un amarrage AG des gaines externes 3, de sorte que les gaines externes 3 des enveloppes 1 ne peuvent plus s'aligner dans le champ de courant induit par le vent.

Cet amarrage AG comprend des liens LG reliant les extrémités distales 31 des gaines externes 3 à un palonnier PL commun perpendiculaire aux gaines externes 3 déployées et flottantes horizontalement sur l'eau, et un point d'amarrage PG, réalisé notamment sous la forme d'une poutre ou tige plantée verticalement dans le fond de l'étendue d'eau, auquel est relié le palonnier PL. Ces liens L comportent avantageusement des articulations rotatives AR, notamment du type émerillon, de façon à ce que les gaines externes 3 puissent tourner librement autour de leurs axes longitudinaux respectifs, ainsi que décrit ci-dessus.

L'ajustement de la longueur des liens LG et leur disposition parallèle permet de répartir les tensions d'amarrage de façon uniforme entre toutes les gaines externes 3 et de permettre leur rotation ou leur oscillation à fin de nettoyer leurs parois externes 31 respectives, ainsi que décrit ci-dessus ; la rotation des gaines externes 3 étant rendue possible par le montage d'articulations rotatives AR sur ces liens LG entre les extrémités distales 31 des gaines externes 3 et le palonnier PL.

Dans le cas où le plan d'eau est navigable, des moyens de signalisation diurnes et nocturnes conformes aux législations locales peuvent équiper les différents points de cet ensemble flottant, et notamment les extrémités distales 31 des gaines externes 3.

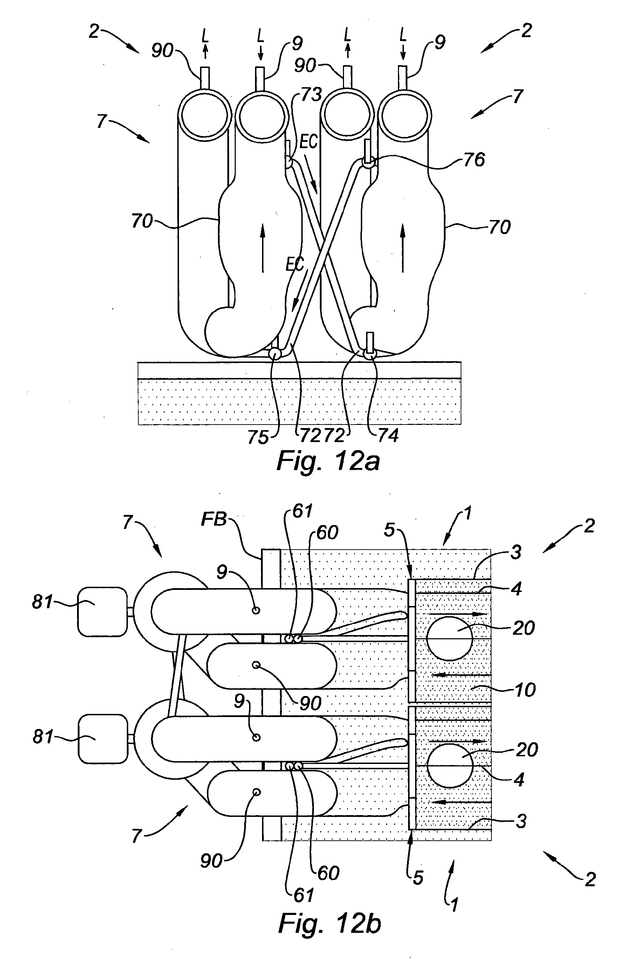

Dans les modes de réalisation illustrés sur les figures 6, 7 et 12, la canalisation de fermeture 7 présente un logement 70 (visible sur les figures 6a, 7, 12, et 13a), de section élargie, destiné à recevoir en partie le moyen de mise en circulation 8. Ce logement 70 s'étend selon une direction principale horizontale, comme visible en figure 6a, ou verticale, comme visible sur les figures 7 et 12.

Dans le mode de réalisation illustré sur les figures 7a à 7d, le moyen de mise en circulation 8 est réalisé sous la forme d'un moyen de propulsion mécanique qui comprend une hélice 80 entraînée en rotation par un moteur rotatif 81 via un arbre de sortie 82 dudit moteur 81. Le moteur 81 est disposé à l'extérieur du réacteur 2 et l'arbre de sortie 82 traverse de façon étanche la canalisation de fermeture 7 pour déboucher à l'intérieur du logement 70 et supporter l'hélice 80 qui est ainsi mobile en rotation à l'intérieur de ce logement 70.

Avantageusement, le logement 70 de l'hélice 80 est disposé entre une zone de divergence et une zone de convergence de la canalisation de fermeture 7 du milieu de culture liquide L, afin d'assurer une continuité hydraulique sans variation brusque de vitesse, ceci dans le but de limiter les pertes de charge, les accélérations et les efforts de cisaillement subis par les microorganismes.

Selon une caractéristique avantageuse et comme illustré sur les figures 7 et 12, le logement 70 est disposé une branche verticale montante de la canalisation de fermeture 7, et donc l'hélice 80 présente un axe vertical de rotation, afin de permettre l'évacuation du gaz G pouvant se former dans le logement 70 et éviter ainsi les phénomènes de cavitation.

De plus, et comme visible sur la figure 7, la disposition du moyen d'échappement 60 de gaz G en amont du moyen de mise en circulation 8, ou de l'hélice 80, conjugué à la disposition du moyen de mise en circulation 8 dans le logement 70 en amont du moyen d'injection de gaz 61 est également avantageuse pour éviter que le gaz ne circule à travers l'hélice 80 et ne nuise à son fonctionnement. En effet, la présence de gaz G gêne le fonctionnement de la plupart des moyens de propulsion mécanique et aux hélices en particulier, et son accumulation doit donc être évitée au risque de faire caviter l'hélice 80.

Dans les modes de réalisation illustrés sur les figures 6, 7 et 11, la canalisation de fermeture 7 présente deux portions recourbées 79, respectivement une portion aller et une portion retour disposée de part et d'autre du logement 70 de réception du moyen de mise en circulation 8. Ces portions recourbées 79, ou portions en col de cygne, présentent une courbure sensiblement à 180° afin de réaliser le franchissement de la paroi ou franc bord FB de l'embarcation E ; un tel franchissement garantissant l'isolement du moyen de mise en circulation 8 vis-à-vis de l'eau et également la sécurité du réacteur et des personnes utilisant ce réacteur.

En outre, et comme illustré sur les figures 6a et 11, ces portions recourbées 79 peuvent être déplaçables en partie dans une position relevée afin de pouvoir sortir de l'eau et relever la platine 5 de raccordement des gaines 3, 4, pour permettre notamment une mise en place des gaines 3, 4 hors de l'eau et sous condition aseptique.

La mise en place des gaines 3, 4 souples est décrite ci-après en référence aux figures 11 a et 11 b. Les gaines 3, 4 peuvent être livrées sous la forme de bobines B, où un flotteur F est positionné au centre de la bobine B pour stabiliser celle-ci en flottation sur l'étendue d'eau.

Comme visible en figure 11a, les deux gaines 3, 4 sont montées sur la platine 5 à leurs extrémités proximales 30, 40 respectives ; ladite platine 5 occupant avantageusement une position relevée comme décrit ci-dessus. Cette opération de fixation des gaines 3, 4 sur la platine 5 se fait aseptiquement pour éviter d'introduire des contaminants dans le milieu de culture.

Comme visible sur la figure 11, la platine 5 est rabaissée et plongée en partie dans l'eau, puis le déploiement des gaines 3, 4 se fait par remplissage à l'aide des milieux liquide L et gazeux G stériles. L'arrivée de liquide et de gaz peut opportunément se faire par toutes les ouvertures 11, 12 ménagées dans la platine 5. En effet, dans le cas contraire, seule la gaine d'arrivée de liquide L et de gaz G reçoit les fluides et subit les efforts de déploiement. Une fois les gaines 3, 4 déroulées et gonflées, la circulation du milieu liquide L peut être établie dans le réacteur 2, par mise en marche du moyen de mise en circulation 8.

Une trappe TR peut être prévue sur l'embarcation E afin d'isoler la partie externe du réacteur 2 de la partie interne de ce réacteur 2 une fois les gaines 3, 4 déroulées et gonflées.

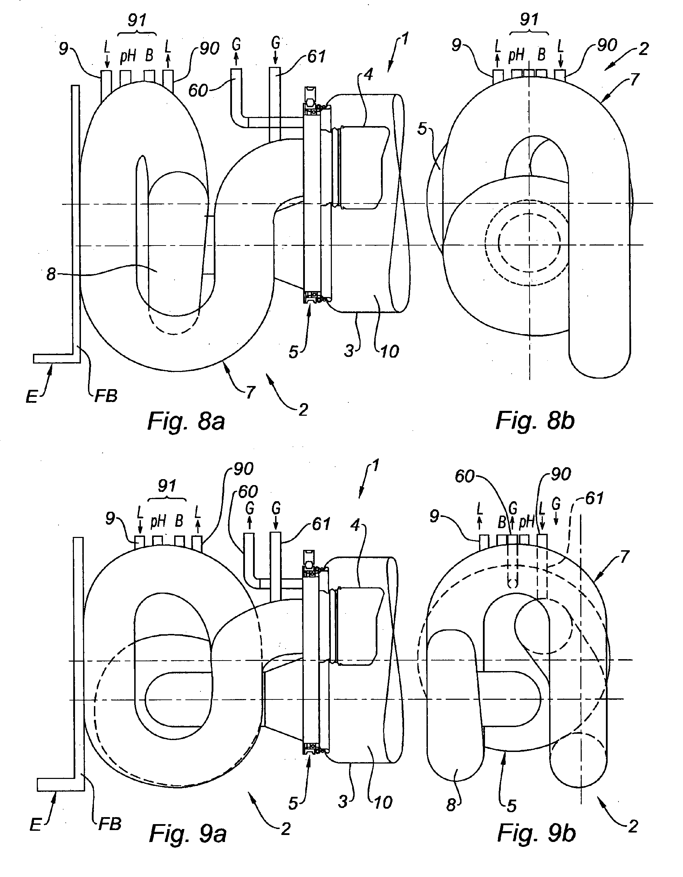

Dans les modes de réalisation illustrés sur les figures 8 et 9, le moyen de mise en circulation est réalisé sous la forme d'une pompe centrifuge 8, l'ensemble étant disposé à l'extérieur de l'embarcation E; on parle alors d'une solution dite hors-bord qui a l'avantage de la compacité mais qui présente l'inconvénient d'être agressive pour les microorganismes. Le passage de corps de nettoyage 20, décrits ci-après, nécessite une canalisation de fermeture 7 présentant une roue ouverte et une volute de gros diamètre qui réduisent l'efficacité. Dans le mode de réalisation illustré en figure 8, la volute est du type volute frontale, et dans le mode de réalisation illustré en figure 9, la volute est du type volute sagitale. Dans ces modes de réalisation avec pompe centrifuge, la platine 5 est solidaire d'une embarcation E ou d'un quai, et peut éventuellement être relevée pour faciliter la mise en place des gaines 3, 4 hors de l'eau.

Comme visible sur les figures 1, 6a, 7a, 7d et 12b, le réacteur 2 peut également comprendre un ou plusieurs corps de nettoyage 20 conformés pour circuler le long du parcours d'écoulement, autrement dit à l'intérieur de la gaine interne 4, de l'espace inter-gaine 10 et de la canalisation de fermeture 7, afin de nettoyer l'intérieur des gaines 3, 4 et de la canalisation de fermeture 7. Pour pouvoir circuler en boucle dans le réacteur 2, le ou les corps de nettoyage 20 sont également conformés pour passer à travers le ou les moyens de mise en circulation 8 du milieu de culture liquide, par exemple à travers les pales de l'hélice 80 dans le mode de réalisation particulier décrit ci-dessus en référence à la figure 7.

Le ou les corps de nettoyage 20, de préférence sphériques, présentent par exemple un diamètre sensiblement égal au diamètre intérieur de la canalisation de fermeture 7 pour optimiser le nettoyage des parois internes de la canalisation de fermeture 7.

Le ou les corps de nettoyage 20 ont également pour but de compléter le nettoyage de la paroi interne 34 de la gaine externe 3 et de la paroi externe 43 de la gaine interne 4 effectué avec le libre mouvement de la gaine interne 4 à l'intérieur de la gaine externe 3 ; ce libre mouvement étant favorisée par la fixation rotative de la gaine externe 3 sur la platine 5.

La différence de vitesses entre la circulation gazeuse et la circulation liquide influe directement sur les transferts de masse gaz/liquide et doit avantageusement être maintenue au niveau le plus élevé possible. C'est la raison pour laquelle chaque corps de nettoyage 20 ne doit pas empêcher le passage du gaz. Pour cela, chaque corps de nettoyage 20 est conformé pour laisser passer au moins partiellement le gaz circulant à l'intérieur de la canalisation de réaction 2 tout en étant adapté pour être entraîné par la circulation du milieu de culture liquide afin que le corps de nettoyage 20 n'influe pas sur la différence de vitesses entre le gaz et le milieu liquide.

A cet effet, le ou chaque corps de nettoyage 20 est réalisé sous la forme d'une brosse, notamment sphérique, comprenant un assemblage de poils, crins, brins ou équivalents, avec une partie centrale porteuse de ces poils. Ainsi, dans les gaines 3, 4 horizontales, les poils émergés laissent passer le gaz au niveau du ciel de gaz et la partie centrale immergée et porteuse des poils présente un diamètre suffisamment grand pour constituer un obstacle au passage du liquide, de sorte que le milieu liquide entraine avec lui le corps de nettoyage 20.

De la même manière, le corps de nettoyage 20 peut être réalisé sous la forme d'une sphère creuse en matière élastomère dont une partie substantielle de la surface est percée de trous qui permettent de laisser passer le gaz.

Le corps de nettoyage 20 doit également présenter une densité supérieure à celle de l'eau, afin de se tenir au contact du plancher des gaines 3, 4 comme illustré sur la figure 1 ; le plancher d'une gaine correspondant à sa partie inférieure ou basse totalement immergée.

Pour éviter tout coincement, le corps de nettoyage 20 spéhrique doit présenter un diamètre Dc n'excédant pas la valeur maximale Dcmax correspondant à la différence des diamètres des gaines 3, 4, ce qui se traduit par la relation R2 suivante : Dcmax = De - Di.

Dans le cas où la relation R1 définie ci-dessus est respectée, on obtient donc : Dcmax = $(2^{1/2} - 1)$.Di.

Du fait de la rotation de la gaine externe 3, la gaine interne 4 à tendance à être entrainée vers le côté plongeant de la gaine externe 3. Il est donc préférable que le diamètre Dc du corps de nettoyage 20 soit sensiblement plus faible que la valeur limite Dcmax définie ci-dessus, pour que le corps de nettoyage 20 puisse se tenir dans le fond de l'espace inter-gaine 10, autrement dit sur le plancher de la gaine externe 3.

En procédant de la sorte, on constate que quelque soit le sens de rotation de la gaine externe 3, le corps de nettoyage 20 est entrainé vers des passages plus larges et non pas plus étroits, ce qui réduit les risques de coincement du corps de nettoyage 20 entre la gaine interne 4 et la gaine externe 3.

Dans un mode de réalisation particulier de l'invention illustré sur les figures 10a et 10b, l'enveloppe 1 comprend en outre :

- des moyens de pincement ou de ligature 13 amovibles conçus pour pincer ou ligaturer les deux gaines 3, 4 sur une zone intermédiaire 16 située entre les extrémités proximales 30, 40 et distales 31, 41 respectives des gaines 3, 4 ;
- au moins un orifice intermédiaire 14 de communication entre l'intérieur de la gaine interne 4 et l'espace inter-gaine 10, ledit orifice intermédiaire 14 étant prévu sur la gaine interne 4 entre son extrémité proximale 40 et la zone intermédiaire 16 de pincement ou de ligature ; et
- des moyens de fermeture 15 de l'orifice intermédiaire 14, notamment du type trappe, lesdits moyens de fermeture 15 étant mobiles entre une position ouverte (illustrée en figure 10a) et une position fermée (illustrée en figure 10b).

Ainsi, la gaine interne 4 comprend une trappe 15 pouvant être manoeuvrée de l'extérieur de la gaine externe 3 pour ouvrir ou fermer cet orifice intermédiaire 14.

La zone intermédiaire 16 de pincement ou de ligature est disposée à environ un dixième de la longueur de la gaine externe 3 ou interne 4, de manière à n'exploiter qu'un sous-volume de réaction, correspondant à la portion de l'enveloppe 1 et des gaines 3, 4 située entre la platine 5 et cette zone intermédiaire 16, et qui peut représenter environ 1/10 du volume total de l'enveloppe 1 ; ce ratio correspondant à celui du volume d'inoculation rapporté au volume de culture retenu classiquement.

Dans un premier temps illustré en figure 10a, les moyens de pincement ou de ligature 13 pincent ou ligaturent les deux gaines 3, 4 sur la zone intermédiaire 16 située au-delà de l'orifice intermédiaire 14, isolant ainsi le sous-volume de réaction. Les gaines 3, 4 sont remplies de milieu nutritif stérile et de gaz et la trappe 15 aménagée dans la gaine interne 4 est ouverte de sorte à laisser le passage au gaz G et au liquide L à l'extrémité de ce sous-volume de réaction. De la sorte, ce sous-volume présente toutes les fonctionnalités du volume entier et peut fonctionner de façon autonome. Il est ainsi possible de l'inoculer et de le mettre en culture.

Dans un second temps illustré en figure 10b, lorsque la concentration atteint un niveau suffisant dans ce sous-volume, les moyens de pincement ou de ligature 13 sont retirés et la trappe 15 est fermée, de telle sorte que le reste du volume est mis en ligne et de ce fait inoculé par le sous-volume ; le réacteur prenant alors sa pleine capacité de production.

Certaines cultures ne doivent pas être exposées à la pleine lumière tant que leur concentration est insuffisante pour les protéger par auto-ombrage. Pour ce type de cultures, il est donc préférable d'assurer une protection contre la luminosité pendant une période de quelques jours après inoculation.

Pour assurer cette protection, une solution consiste à recouvrir la gaine externe 3 d'une bande d'ombrage (non illustrée) longitudinale réalisée dans un matériau opaque ou présentant une forte absorbance de la lumière. Ainsi, la luminosité est fortement atténuée à l'intérieur de l'enveloppe 1 lorsque la bande d'ombrage est interposée entre la culture et le soleil.

Deux modes de réalisation de la bande d'ombrage sont envisageables, avec dans un premier mode de réalisation une première bande d'ombrage et dans un second mode de réalisation une seconde bande d'ombrage.

Dans la première réalisation, la première bande d'ombrage est rapportée et positionnée temporairement sur la gaine externe 3, et plus spécifiquement sur la partie émergée (hors de l'eau) de la gaine externe 3. La première bande d'ombrage peut recouvrir totalement la surface émergée de cette gaine externe 3 au-dessus de la ligne de flottaison, et occupe par exemple entre un quart et un tiers du périmètre totale de la gaine externe 3 (suivant la position de la ligne de flottaison).

Cette première bande d'ombrage est positionnée de façon fixe sur la gaine externe 3 pendant les premiers jours de culture où la rotation de la gaine externe 3 est interrompue. Une fois que la concentration de la culture atteint la valeur seuil définie pour l'espèce cultivée, la rotation de la gaine extrerne 3 est établie et la première bande d'ombrage peut être retirée.

Dans la seconde réalisation, la seconde bande d'ombrage est fixée solidairement sur la gaine externe 3, et ainsi est intégrée directement sur la gaine externe 3. La seconde bande d'ombrage peut être fixée sur la gaine externe 3 par collage ou soudage, ou en variante la seconde bande d'ombrage peut se présenter sous la forme d'une couche de matière (par exemple un film de peinture ou un film de matériau opaque) déposée fixement sur la gaine externe 3. Cette seconde bande d'ombrage occupe par exemple entre un quart et un tiers du périmètre totale de la gaine externe 3.

Cette seconde bande d'ombrage peut recouvrir totalement la surface émergée de cette gaine externe 3, au-dessus de la ligne de flottaison, pendant les premiers jours de culture où la rotation de la gaine externe 3 est interrompue. Une fois que la concentration de la culture atteint la valeur seuil définie pour l'espèce cultivée, la rotation de la gaine extrerne 3 est établie et la seconde bande d'ombrage reste en place sur la gaine externe 3.

Pour éviter que cette rotation ne fasse passer la seconde bande d'ombrage entre le soleil et la culture, ce qui se traduirait par un manque à produire, la rotation peut débuter avec un demi-tour qui amène la seconde bande d'ombrage en partie inférieure immergée (autrement dit sous la ligne de flottaison, au niveau du plancher de la gaine externe 3). Ensuite, la rotation est poursuivie mais de façon incomplète (c'est-à-dire pas sur un tour complet, et même pas sur un demi-tour) et organisée de façon alternative dans un sens puis dans l'autre, afin de maintenir constamment la seconde bande d'ombrage dans la partie inférieure immergée de la gaine externe 3 par laquelle passe moins de lumière. Dans ce cas, il est nécessaire d'établir un compromis entre le nettoyage des gaines 3, 4 et l'exposition à la lumière, car une rotation partielle ne nettoie qu'une partie de la circonférence de la gaine externe 3, tandis qu'une rotation complète diminue la production par atténuation répétée de la lumière du fait de la présence de la seconde bande d'ombrage. Une rotation partielle sur les trois quarts ou les deux tiers de la circonférence pourrait constituer une solution de compromis avantageuse.

Comme illustré sur les figures 12a et 12b, l'invention concerne également un ensemble de réacteurs photosynthétiques comprenant au moins deux réacteurs 2 conformes à l'invention, à savoir un premier (à gauche) et un deuxième (à droite) réacteurs, et comprenant au moins une canalisation de liaison 71, 72 assurant une liaison fluidique entre le premier réacteur et le deuxième réacteur et au moins une vanne 77, 78 disposée sur ladite canalisation de liaison 71, 72, afin de permettre l'inoculation d'un réacteur par l'autre réacteur. Il est ainsi possible d'interconnecter deux réacteurs de telle sorte que leurs contenus soient échangés, afin de rendre possible l'inoculation d'un réacteur par un autre dont la concentration aura atteint un stade avancé.

Dans le mode de réalisation illustré sur les figures 12a et 12b, l'ensemble comprend deux canalisations de liaison 71, 72 entre les deux réacteurs 2. Les canalisations de liaison 71, 72 sont pourvues, à leurs extrémités respectives, de vannes respectivement 73, 74 pour la canalisation de liaison 71 et respectivement 75, 76 pour la canalisation de liaison 72.

La première canalisation de liaison 71 relie un point d'entrée disposé sur le premier réacteur 2 en aval du logement 70 de réception du moyen de mise en circulation, tel que l'hélice rotative (non visible), à un point de sortie disposé sur le deuxième réacteur en amont du logement 70 de réception du moyen de mise en circulation de ce deuxième réacteur 2.

La deuxième canalisation de liaison 72 relie un point d'entrée disposé sur le deuxième réacteur 2 en aval du logement 70 de réception du moyen de mise en circulation, tel que l'hélice rotative (non visible), à un point de sortie disposé sur le premier réacteur en amont du logement 70 de réception du moyen de mise en circulation de ce deuxième réacteur 2

Les réacteurs 2 sont assemblés de façon parallèle pour constituer un ensemble productif cohérent. Afin de rendre possible l'inoculation d'un réacteur par son voisin dont la concentration en microorganismes aurait atteint un stade avancé, l'ensemble prévoit d'interconnecter ces deux réacteurs avec les canalisations de liaison 71, 72 de telle sorte que leurs contenus respectifs soient mélangés.

En outre, comme visible sur la figure 12a, les points de sortie des canalisations de liaison 71, 72 sont placés à l'extrémité des zones de convergence situées en amont des logements 70 correspondants pour bénéficier d'un effet Venturi.

Les vannes 73, 74, 75, 76 permettent la connexion sous asepsie des deux canalisations de liaison 71, 72 qui relient de façon croisée et symétrique les points d'entrée et les points de sortie des deux réacteurs 2 à interconnecter. Les vannes 73, 74, 75, 76 sont disposées sensiblement aux points d'entrée et de sortie des canalisations de liaison 71, 72 correspondantes.

L'utilisation d'un tel ensemble peut se faire de la manière suivante pour procéder à l'inoculation du deuxième réacteur 2 (à droite) à partir du premier réacteur 2 (à gauche) déjà en service lorsque la concentration en microorganismes a atteint le niveau d'exploitation.

Dans un premier temps, les vannes 73, 74 et leurs vis-à-vis 75, 76 sont fermées, le premier réacteur 2 est en service avec l'établissement de la circulation à l'intérieur de ce premier réacteur, et le deuxième réacteur 2 à inoculer est rempli de milieu nutritif stérile.

Dans un deuxième temps, la circulation est établie à l'intérieur du deuxième réacteur 1 B et les vannes 73, 74 et leurs vis à vis 75, 76 sont ouvertes pour établir un échange croisé entre les deux réacteurs comme illustré par les flèches EC de la figure 12a.

Après l'ouverure des vannes 73, 74 et leurs vis à vis 75, 76, les concentrations deviennent sensiblement égales dans les deux réacteurs 2 et il est possible de les isoler par fermeture des vannes 73, 74 et leurs vis à vis 75, 76. Pour réduire la durée de cet échange, une pompe (non visible) peut être interposée sur l'une et/ou l'autre des canalisations de liaison 71, 72.

Comme illustré sur les figures 13a et 13b, l'invention concerne également un réacteur 2 comportant une pluralité d'enveloppes 1 de réaction raccordées de façon parallèle sur des platines 5 de raccordement, ce réacteur 2 comprenant une seule et même canalisation de fermeture 7 présentant un logement 70 de réception d'un moyen de mise en circulation 8, tel qu'une système hélice 80/moteur rotatif 81, et une pluralité de conduites en liaison fluidique avec les enveloppes 1.

Dans cette forme particulière de réalisation, qui consiste à interconnecter des enveloppes 1 de réaction en parallèle, l'intérêt est de mettre en commun certaines fonctionnalités, comme les moyens de mise en circulation les moyens de régulation, mais avec l'inconvénient d'accroître l'exposition aux accidents, et notamment aux contaminations.

Dans cette réalisation, la canalisation de fermeture 7 comporte une conduite de collecte 66 dans laquelle est prévue le logement 70 de réception du moyen de mise en circulation 8, et une pluralité de conduites de distribution 67 connectées, d'une part, à la conduite de collecte 66 et, d'autre part, aux enveloppes 1 respectives, de sorte que le milieu liquide est collecté en sortie des enveloppes 1, passe dans le moyen de mise en circulation, puis est distribué à l'entrée des enveloppes 1. Opportunément, la distribution se fait dans le même ordre que la collecte afin d'uniformiser les débits dans les enveloppes 1.

Opportunément, et afin de maintenir les vitesses aussi uniformes que possible; la conduite de collecte 66 et les conduites de distribution 67 présentent une section variable, allant en diminuant d'une extrémité à l'autre de la canalisation qu'ils constituent.

Le procédé de culture de microorganismes photosynthétiques, notamment d'algues, utilisant un réacteur 2 conforme à l'invention, comprend les étapes suivantes :

- injection d'un milieu de culture liquide dans l'enveloppe 1 de réaction selon un débit contrôlé avec le moyen d'injection 9 de liquide ;
- injection d'un gaz G dans l'enveloppe 1 de réaction selon un débit contrôlé avec le moyen d'injection 61 de gaz ;
- mise en pression de la gaine externe 3 de l'enveloppe 1 consistant à créer une surpression de gonflement dans cette gaine externe 3 pour assurer la flottabilité de cette gaine externe 3 et son déploiement ;
- mise en circulation du milieu de culture liquide avec le moyen de mise en circulation 8 ;
- contrôle du moyen de mise en circulation 8 et du moyen d'injection 61 de gaz pour établir dans l'enveloppe 1 de réaction un régime d'écoulement diphasique gaz / milieu de culture liquide du type écoulement stratifié ou écoulement à poches ou à bulles allongées ; et

- récupération des microorganismes photosynthétiques avec la canalisation de sortie 90.

Pendant le parcours dans l'enveloppe 1, le milieu liquide contenant les microorganismes photosynthétiques reçoit le rayonnement solaire à travers la matière transparente des gaines 3, 4, échange de la chaleur avec le plan d'eau par diffusion, par mélange et par conduction à travers cette même matière, et échange des composants avec le gaz G à travers leur interface commune. La capacité de production dépend surtout de la longueur des gaines 3, 4 de l'enveloppe 1 ; plusieurs enveloppes 1 pouvant utilement être disposées en nombre les unes à côté des autres pour assurer la fonction de nettoyage essentielle décrite ci-dessus.

De façon avantageuse, la vitesse de circulation du gaz est établie entre environ 0,5 et 1,5 m/s, correspondant à un régime de vitesses adéquat pour les débits nécessaires à la réaction.

De façon encore avantageuse, le moyen de mise en circulation 8 comprend une hélice 80 entraînée en rotation par un moteur 81 et la vitesse de rotation de l'hélice 80 est inférieure à environ 100 tours par minute, afin de limiter les sollicitations mécaniques au sein du milieu de culture liquide.

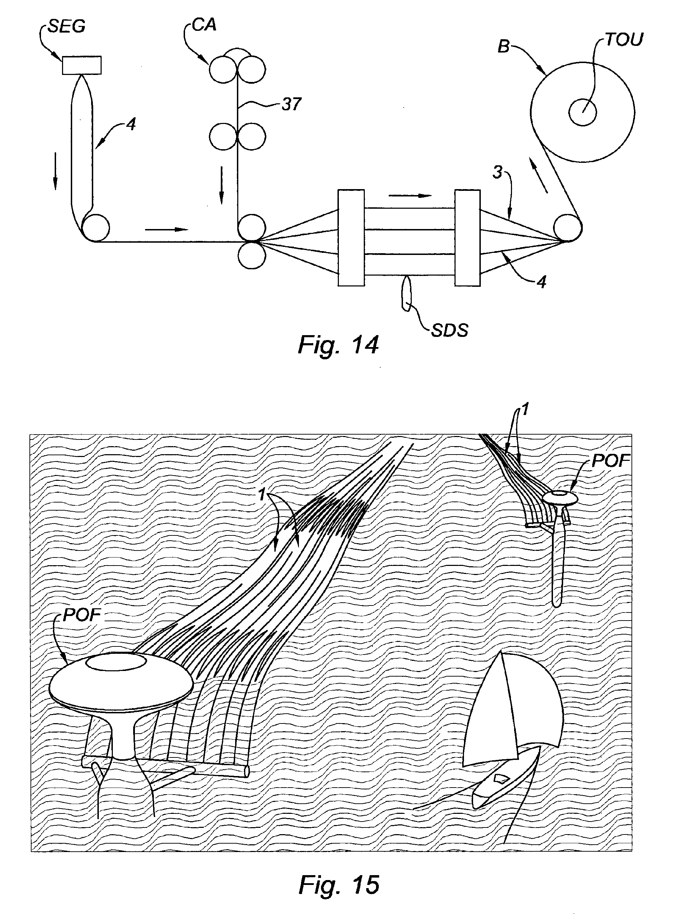

L'invention concerne également un procédé de fabrication d'une enveloppe 1, décrit en référence à la figure 14, comprenant les étapes suivantes :

- confection de la gaine interne 4 par un procédé d'extrusion d'une matière plastique et de gonflage du plastique extrudé, autrement appelé procédé d'extrusion gonflage utilisant un système d'extrusion gonflage SEG ;
- confection d'une nappe externe 37 en matière plastique, notamment par un procédé de calandrage utilisant une calandre CA ;
- enveloppement de la gaine interne 4 par la nappe externe 37 jusqu'à une jonction de deux bords longitudinaux opposés de la nappe externe 37 ;
- soudure de la nappe externe 37 le long de ses deux bords opposés joints lors de l'étape d'enveloppement, au moyen d'un système de soudure SDS, de manière à former la gaine externe 3 entourant la gaine interne 4 ; et
- enroulement des deux gaines 3, 4 l'une dans l'autre sur un touret TOU unique, de manière à former une bobine B.

Les gaines 3, 4 sont produites et disposées l'une dans l'autre en atelier et avant livraison. Ce procédé répond à la problématique d'introduction d'une gaine de grande longueur dans une autre en proposant une fabrication simultanée et continue de ces deux gaines 3, 4, de préférence de façon aseptique afin de réduire les risques de contamination initiale des cultures.

Ce procédé de fabrication qui consiste à fermer la gaine externe 3 autour de la gaine interne 4 permet d'équiper cette gaine interne 4 en cours d'assemblage, notamment de l'orifice de communication 42 et éventuellement de l'orifice intermédiaire 14 muni de sa trappe 15.

L'enroulement des deux gaines 3, 4 disposées l'une dans l'autre commence par leurs extrémités distales avant le déploiement ou déroulement décrit ci-dessus en référence aux figures 11a et 11b. La gaine externe 4 est fermée à son extrémité distale, comme décrit ci-dessus, et cette extrémité distale est éventuellement équipée d'un système d'accrochage d'un lien, tel que le lien LG décrit ci-dessus en référence à la figure 5.

Opportunément, l'épaisseur du film constitutif des gaines 3, 4 peut évoluer depuis l'extrémité distale jusqu'à l'extrémité proximale pour accroître la résistance en même temps que les efforts s'exerçant sur le film.

Bien entendu l'exemple de mise en oeuvre évoqué ci-dessus ne présente aucun caractère limitatif et d'autres améliorations et détails peuvent être apportés à l'enveloppe, au réacteur et aux procédés selon l'invention, sans pour autant sortir du cadre de l'invention où d'autres formes de gaine externe et/ou de gaine interne et/ou de platine de raccordement et/ou de canalisation de fermeture peuvent par exemple être réalisées.

Il est ainsi envisageable, comme illustré schématiquement sur la figure 15, de prévoir l'amarrage des enveloppes 1 sur une plateforme offshore POF en haute mer. Cette plateforme offshore POF constitue une structure flottante de service qui peut par exemple accueillir un équipage et donc le fonctionnement est de préférence entièrement automatisé.

La plateforme offshore POF est de préférence constituée par une bouée de type perche de Froude, très peu sensible à l'agitation de surface. Dans ce cas, la partie immergée de la bouée contient les réserves de flottabilité en partie haute, le lest en partie basse et les fonctionnalités et stockages aux niveaux intermédiaires.

Sur cette plateforme offshore POF peut également être prévue une structure portant les platines de raccordement des gaines des enveloppes 1, qui est reliée à la plateforme offshore POF par un ou deux bras dont l'inclinaison est réglable ; cette structure portant les platines de raccordement pouvant ainsi être remontée en surface pour intervenir sur les platines ou immergée en mode normal de fonctionnement.

D'autres activités, telles que l'élevage de poissons, peuvent être associés à cette plateforme offshore POF. Des nasses de confinement des poissons d'élevage peuvent par exemple être placées sous la nappe des enveloppes 1 flottant sur la surface de la mer.

En outre, il est également envisageable que la plateforme offshore POF soit munie de moyens de propulsion, éventuellement automatisés, afin que celle-ci se déplace pour suivre les températures de surface les plus adaptées à la culture des microorganismes photosynthétiques, échapper aux conditions météorologiques les plus défavorables, et éviter les collisions. Les déplacements de la plateforme offshore POF sont contrôlés in situ ou depuis la terre en fonction des informations météo et radar pour rechercher les meilleures routes. La plateforme offshore POF peut également se rapprocher des côtes pour décharger sa production et pour être ravitaillée.

**Revendications**

1. Enveloppe (1) de réaction pour un réacteur (2) photosynthétique adapté pour la culture de microorganismes photosynthétiques, notamment d'algues, ladite enveloppe (1) de réaction étant conçue pour, d'une part, flotter sur une étendue d'eau et, d'autre part, délimiter un parcours d'écoulement diphasique gaz / milieu de culture liquide entre une première (11) et une seconde (12) ouvertures de ladite enveloppe (1) de réaction, ladite enveloppe (1) de réaction étant **caractérisée en ce qu'**elle comporte deux gaines, respectivement externe (3) et interne (4), réalisées au moins partiellement dans un matériau transparent au rayonnement lumineux, la gaine interne (4) s'étendant à l'intérieur de la gaine externe (3) de sorte que lesdites gaines délimitent entre elles un espace inter-gaine (10) en liaison fluidique avec la première ouverture (11) de l'enveloppe (1) de réaction, **en ce que** la gaine externe (3) présente une extrémité proximale (30) ouverte et une extrémité distale (31) fermée, et **en ce que** la gaine interne (4) présente une extrémité proximale (40) ouverte en liaison fluidique avec la seconde ouverture (12) de l'enveloppe (1) de réaction et une extrémité distale (41) pourvue d'au moins un orifice de communication (42) entre l'intérieur de la gaine interne (4) et l'espace inter-gaine (10).

2. Enveloppe (1) selon la revendication 1, dans laquelle l'une au moins des deux gaines, respectivement externe (3) et interne (4), est réalisée dans un matériau souple adapté pour permettre le pliage, le gonflage, la déformation transversale et/ou le fléchissement de ladite gaine.

3. Enveloppe (1) selon les revendications 1 ou 2, comprenant en outre :

   - une pièce externe de raccordement (50) sur laquelle est montée hermétiquement l'extrémité proximale (30) de la gaine externe (3), et sur laquelle est ménagée la première ouverture (11) de l'enveloppe (1) de réaction en liaison fluidique avec l'espace inter-gaine (10) ; et
   - une pièce interne de raccordement (51) sur laquelle est montée hermétiquement l'extrémité proximale (40) de la gaine interne (4), et sur laquelle est ménagée la seconde ouverture (12) de l'enveloppe (1) de réaction en liaison fluidique avec l'extrémité proximale (40) de la gaine interne (4).

4. Enveloppe (1) selon la revendication 3, dans laquelle l'extrémité proximale (40) de la gaine interne (4) est montée rotative sur la pièce interne de raccordement (51).

5. Enveloppe (1) selon les revendications 3 ou 4, dans laquelle la pièce interne de raccordement (51) est montée à l'intérieur de la pièce externe de raccordement (50).

6. Enveloppe (1) selon l'une quelconque des revendications 3 à 5, dans laquelle la pièce externe de raccordement (50) comporte des moyens de couplage (52) avec des moyens d'entraînement (53) en rotation de ladite pièce externe de raccordement (50) afin d'entraîner en rotation la gaine externe (3).

7. Enveloppe (1) les revendications 5 et 6, dans laquelle la pièce interne de raccordement (51) est libre en rotation dans la pièce externe de raccordement (50), afin que la rotation la gaine externe (3) assure la rotation de la gaine interne (4) par frottement entre les deux gaines.

8. Enveloppe (1) selon l'une quelconque des revendications précédentes, comprenant en outre une troisième gaine en matériau souple s'étendant à l'intérieur de la gaine interne (4) afin de permettre une injection ou une aspiration de gaz aux extrémités distales (31, 41) des deux gaines, respectivement externe (3) et interne (4).

9. Enveloppe (1) selon l'une quelconque des revendications précédentes en combinaison avec la revendication 2, comprenant en outre :

   - des moyens de pincement ou de ligature (13) amovibles conçus pour pincer ou ligaturer les deux gaines (3, 4) sur une zone intermédiaire (16) située entre les extrémités proximales (30, 40) et distales (31, 41) respectives des deux gaines ;
   - au moins un orifice intermédiaire (14) de communication entre l'intérieur de la gaine interne (4) et l'espace inter-gaine (10), ledit orifice intermédiaire (14) de communication étant prévu sur la gaine interne (4) entre son extrémité proximale (30) et ladite zone intermédiaire (16) de pincement ou de ligature ; et
   - des moyens de fermeture (15) dudit orifice intermédiaire (14) de communication, notamment du type trappe, lesdits moyens de fermeture (15) étant mobiles entre une position ouverte et une position fermée.

**10.** Enveloppe (1) selon l'une quelconque des revendications précédentes, comportant en outre une bande d'ombrage longitudinale recouvrant partiellement la gaine externe (3) et réalisée dans un matériau opaque ou présentant une forte absorbance de la lumière, la bande d'ombrage étant notamment rapportée et positionnée temporairement sur la gaine externe (3) ou fixée solidairement sur la gaine externe (3).

**11.** Réacteur (2) photosynthétique adapté pour la culture de microorganismes photosynthétiques, notamment d'algues, comportant :

- au moins une enveloppe (1) de réaction conforme à l'une quelconque des revendications précédentes ;
- au moins une canalisation de fermeture (7) assurant la liaison fluidique entre la première (11) et la seconde (12) ouvertures de ladite enveloppe (1) de réaction ;
- au moins un moyen de mise en circulation (8) disposé dans ladite canalisation de fermeture (7) et conçu pour mettre en circulation le milieu de culture liquide dans la canalisation de fermeture (7) et dans l'enveloppe (1) de réaction ;
- au moins un moyen d'injection (9) de liquide disposé dans ladite canalisation de fermeture (7) et conçu pour permettre d'injecter du liquide dans l'enveloppe (1) de réaction ;
- au moins un moyen d'injection (61) de gaz disposé dans ladite canalisation de fermeture (7) et conçu pour permettre d'injecter du gaz dans l'enveloppe (1) de réaction ; et
- au moins un moyen d'échappement (60) de gaz disposé dans ladite canalisation de fermeture (7) et conçu pour permettre l'échappement du gaz injecté dans l'enveloppe (1) de réaction.

**12.** Procédé de culture de microorganismes photosynthétiques, notamment d'algues, utilisant un réacteur conforme à la revendication 11 et comprenant les étapes suivantes :

- injection d'un milieu de culture liquide dans l'enveloppe (1) de réaction selon un débit contrôlé avec le moyen d'injection (9) de liquide ;
- injection d'un gaz dans l'enveloppe (1) de réaction selon un débit contrôlé avec le moyen d'injection (61) de gaz ;
- mise en circulation du milieu de culture liquide avec le moyen de mise en circulation (8) ;
- contrôle du moyen de mise en circulation (8) et du moyen d'injection (61) de gaz pour établir dans l'enveloppe (1) de réaction un régime d'écoulement diphasique gaz / milieu de culture liquide du type écoulement stratifié ou écoulement à poches ou à bulles allongées.

**13.** Procédé selon la revendication 12 dans lequel l'étape de contrôle comprend une étape de contrôle de la vitesse de circulation du liquide dans l'enveloppe (1) de réaction entre environ 0,1 et 1,0 m/s.

**14.** Procédé selon les revendications 12 ou 13, dans lequel le moyen de mise en circulation (8) comprend une hélice (80) entraînée en rotation par un moteur (81), et dans lequel la vitesse de rotation de l'hélice est inférieure à environ 1000 tours par minute, préférentiellement inférieure à environ 100 tours par minute.

**15.** Procédé de fabrication d'une enveloppe (1) de réaction conforme à l'une quelconque des revendications 1 à 10, comprenant les étapes suivantes :

- confection de la gaine interne (4), notamment par un procédé d'extrusion d'une matière plastique et de gonflage du plastique extrudé ;
- confection d'une nappe externe (37) en matière plastique, notamment par un procédé de calandrage ;
- enveloppement de la gaine interne (4) par la nappe externe (37) jusqu'à une jonction de deux bords opposés ;
- soudure de la nappe externe (37) le long de ses deux bords opposés joints lors de l'étape d'enveloppement, de manière à former la gaine externe (3) entourant la gaine interne (4).

**Patentansprüche**

**1.** Reaktionshülle (1) für einen Photosynthesereaktor (2) für die Kultur photosynthetischer Mikroorganismen, im Speziellen von Algen, wobei die besagte Reaktionshülle (1) einerseits dafür konzipiert wurde, auf einer Wasserfläche zu schwimmen, und andererseits, um einen Zwei-Phasen-Strömungspfad für Gas/flüssiges Nährmedium zwischen einer ersten (11) und einer zweiten (12) Öffnung der besagten Reaktionshülle (1) einzugrenzen, wobei die besagte Reaktionshülle (1) **dadurch gekennzeichnet ist, dass** sie zwei Mäntel, jeweils einen äußeren (3) und einen inneren (4) Mantel umfasst, die zumindest teilweise aus einem für Lichtstrahlen transparenten Material gefertigt sind, wobei sich der innere Mantel (4) im Inneren des äußeren Mantels (3) erstreckt, sodass die besagten Mäntel zwischen sich einen Zwischenmantelraum (10) mit einer Fluidverbindung mit der ersten Öffnung (11) der Reaktionshülle (1) eingrenzen, dadurch, dass der äußere Mantel (3) ein offenes proximales Ende (30) und ein geschlossenes distales Ende (31) aufweist, und dadurch, dass der innere Mantel (4) ein offenes proximales Ende (40) mit einer Fluidverbindung mit der zweiten Öffnung (12) der Reaktionshülle (1) und ein distales Ende (41) aufweist, das zumindest mit einer Verbindungsöffnung (42) zwischen dem Inneren des inneren Mantels (4) und dem Zwischenmantelraum (10) versehen ist.

2. Hülle (1) nach Anspruch 1 bei der zumindest einer der beiden Mäntel, jeweils der äußere (3) und der innere (4) Mantel, aus einem flexiblen Material gefertigt ist, das sich dazu eignet, um den besagten Mantel falten, aufblasen, in Querrichtung verformen und/oder biegen zu können.

3. Hülle (1) nach den Ansprüchen 1 oder 2, die darüber hinaus folgendes umfasst:

    - ein externes Anschlussstück (50) an dem das proximale Ende (30) der äußeren Hülle (3) hermetisch dicht montiert ist, und auf dem die erste Öffnung (11) der Reaktionshülle (1) mit einer Fluidverbindung mit dem Zwischenmantelraum (10) angeordnet ist; und
    - ein internes Anschlussstück (51), an dem das proximale Ende (40) des inneren Mantels (4) hermetisch dicht montiert ist, und auf dem die zweite Öffnung (12) der Reaktionshülle (1) mit einer Fluidverbindung mit dem proximalen Ende (40) des inneren Mantels (4) angeordnet ist.

4. Hülle (1) nach Anspruch 3, bei der das proximale Ende (40) des inneren Mantels (4) drehend auf dem internen Anschlussstück (51) montiert ist.

5. Hülle (1) nach den Ansprüchen 3 oder 4, bei der das interne Anschlussstück (51) im Inneren des externen Anschlussstücks (50) montiert ist.

6. Hülle (1) nach irgendeinem der Ansprüche 3 bis 5, bei der das externe Anschlussstück (50) Koppelvorrichtungen (52) mit Vorrichtungen (53) enthält, die das besagte externe Anschlussstück (50) in Drehung versetzen, um den äußeren Mantel (3) in Drehung zu versetzen.

7. Hülle (1) nach den Ansprüchen 5 und 6, bei der das interne Anschlussstück (51) im externen Anschlussstück (50) frei drehen kann, sodass die Drehung des äußeren Mantels (3) den inneren Mantel (4) durch die Reibung zwischen den beiden Mänteln in Drehung versetzt.

8. Hülle (1) nach irgendeinem der vorherigen Ansprüche, die darüber hinaus einen dritten Mantel aus einem flexiblen Material umfasst, der sich im Inneren des inneren Mantels (4) erstreckt, um die Injektion oder die Extraktion von Gas an den distalen Enden (31, 41) der beiden Mäntel, jeweils des äußeren (3) und des inneren (4) Mantels, zu ermöglichen.

9. Hülle (1) nach irgendeinem der vorherigen Ansprüche in Verbindung mit Anspruch 2, die darüber hinaus folgendes umfasst:

    - abnehmbare Klemm- oder Ligaturvorrichtungen (13), die gestaltet wurden, um die beiden Mäntel (3, 4) auf einem Zwischenbereich (16) zu klemmen oder zu verknüpfen, der sich zwischen den jeweiligen proximalen (30, 40) und distalen (31, 41) Enden der beiden Mäntel befindet;
    - zumindest eine Zwischenverbindungsöffnung (14) zwischen dem Inneren des inneren Mantels (4) und dem Zwischenmantelraum (10), wobei die besagte Zwischenverbindungsöffnung (14) auf dem inneren Mantel (4) zwischen seinem proximalen Ende (30) und dem besagten Zwischenklemm- oder Ligaturbereich (16) angeordnet ist; und
    - Vorrichtungen (15) zum Verschließen der besagten Zwischenverbindungsöffnung (14), im Speziellen in der Art einer Klappe, wobei die besagten Verschließvorrichtungen (15) zwischen einer offenen Stellung und einer geschlossenen Stellung bewegt werden können.

10. Hülle (1) nach irgendeinem der vorherigen Ansprüche, die darüber hinaus ein Längsschattenband umfasst, das den äußeren Mantel (3) teilweise abdeckt, und aus einem Material gefertigt ist, welches undurchsichtig oder stark lichtabsorbierend ist, wobei das Schattenband im Speziellen vorübergehend auf dem äußeren Mantel (3) aufgebracht und positioniert, oder fest auf dem äußeren Mantel (3) befestigt wird.

11. Photosynthesereaktor (2), der sich für die Kultur photosynthetischer Mikroorganismen, im Speziellen von Algen, eignet, folgendes umfassend:

    - zumindest eine Reaktionshülle (1) nach irgendeinem der vorherigen Ansprüche;
    - zumindest eine Rücklaufleitung (7) für die Fluidverbindung zwischen der ersten (11) und der zweiten (12) Öffnung der besagten Reaktionshülle (1);
    - zumindest eine Zirkulationsvorrichtung (8), die in der besagten Rücklaufleitung (7) angeordnet, und gestaltet ist, um das flüssige Kulturmedium in der Rücklaufleitung (7) und in der Reaktionshülle (1) zum Zirkulieren zu bringen;
    - zumindest eine Flüssigkeitsinjektionsvorrichtung (9), die in der besagten Rücklaufleitung (7) angeordnet, und gestaltet ist, um die Injektion der Flüssigkeit in die Reaktionshülle (1) zu ermöglichen;
    - zumindest eine Gasinjektionsvorrichtung (61), die in der besagten Rücklaufleitung (7) angeordnet, und gestaltet ist, um die Injektion des Gases in die Reaktionshülle (1) zu ermöglichen; und

- zumindest eine Gasauslassvorrichtung (60), die in der besagten Rücklaufleitung (7) angeordnet, und gestaltet ist, um das Abführen des in die Reaktionshülle (1) injizierten Gases zu ermöglichen.

12. Verfahren zur Kultur photosynthetischer Mikroorganismen, im Speziellen von Algen, unter Verwendung eines Reaktors nach Anspruch 11, und die folgenden Schritte umfassend:

- Injektion eines flüssigen Kulturmediums in die Reaktionshülle (1) gemäß einer kontrollierten Flussrate unter Verwendung der Flüssigkeitsinjektionsvorrichtung (9);
- Injektion eines Gases in die Reaktionshülle (1) gemäß einer kontrollierten Flussrate unter Verwendung der Gasinjektionsvorrichtung (61);
- Zirkulieren lassen des flüssigen Kulturmediums anhand der Zirkulationsvorrichtung (8);
- Kontrolle der Zirkulationsvorrichtung (8) und der Gasinjektionsvorrichtung (61), um in der Reaktionshülle (1) ein Zwei-Phasen-Strömungsprofil mit Gas/ flüssigem Nährmedium in der Art einer geschichteten, oder taschenförmigen Strömung oder einer Strömung mit gestreckten Blasen zu erzeugen.

13. Verfahren nach Anspruch 12, bei dem der Kontrollschritt einen Schritt zur Kontrolle der Zirkulationsrate der Flüssigkeit in der Reaktionshülle (1) zwischen etwa 0,1 und 1,0 m/s umfasst.

14. Verfahren nach den Ansprüchen 12 oder 13, bei der die Zirkulationsvorrichtung (8) einen Propeller (80) umfasst, der durch einen Motor (81) in Drehung versetzt wird, und bei dem die Drehzahl des Propellers geringer als etwa 1000 U/min., und vorzugsweise geringer als etwa 100 U/min. ist.

15. Verfahren zur Herstellung einer Reaktionshülle (1) nach irgendeinem der Ansprüche 1 bis 10, die folgenden Schritte umfassend:

- Herstellung des inneren Mantels (4), im Speziellen durch Extrudieren eines Kunststoffmaterials und Aufblasen des extrudierten Kunststoffs;
- Herstellung einer äußeren Decke (37) aus Kunststoffmaterial, im Speziellen durch Kalandrieren;
- Umhüllen des inneren Mantels (4) mit der äußeren Decke (37), bis die beiden gegenüber liegenden Ränder einander berühren; und
- Verschweißen der äußeren Decke (37) entlang der beiden im Hüllschritt aneinander gelegten gegenüberliegenden Ränder, sodass der äußere Mantel (3) gebildet wird, der den inneren Mantel (4) umgibt.

**Claims**

1. A reaction casing (1) for photosynthetic reactor (2) for the culture of photosynthetic microorganisms, algae in particular, the said reaction casing (1) being designed firstly to float on a body of water and secondly to delimit a biphasic flow pathway for the gas/liquid culture medium between a first (11) and a second (12) opening of the said reaction casing (1), the said reaction casing (1) being **characterized in that** it comprises two claddings, respectively outer (3) and inner (4), made at least in part of a material transparent to light rays, the inner cladding (4) extending inside the outer cladding (3) so that the said claddings delimit between them an inter-cladding space (10) in fluid connection with the first opening (11) of the reaction casing (1), **in that** the outer cladding (3) has an open proximal end (30) and a closed distal end (31) and **in that** the inner cladding (4) has an open proximal end (40) in fluid connection with the second opening (12) of the reaction casing (1) and a distal end (41) provided with at least one communication orifice (42) between the inside of the inner cladding (4) and the inter-cladding space (10).

2. The casing (1) according to claim 1 wherein at least one of the two claddings, respectively outer (3) and inner (4), is made in a flexible material adapted to allow the folding, inflation, transverse deformation and/or bending of the said cladding.

3. The casing (1) according to claims 1 or 2 further comprising:

- an external connection part (50) on which the proximal end (30) of the outer cladding (3) is hermetically mounted, and on which the first opening (11) of the reaction casing (1) is arranged in fluid connection with the inter-cladding space (10); and
- an internal connection part (51) on which the proximal end (40) of the inner cladding (4) is hermetically mounted and on which the second opening (12) of the reaction casing (1) is arranged in fluid connection with the proximal end (40) of the inner cladding (4).

4. The casing (1) according to claim 3 wherein the proximal end (40) of the inner cladding (4) is mounted in rotation on the internal connection part (51).

5. The casing (1) according to claims 3 or 4 wherein the internal connection part (51) is mounted inside the external connection part (50).

6. The casing (1) according to any of claims 3 to 5 wherein the external connection part (50) comprises coupling means (52) with means (53) for driving in rotation the said external connection part (50) to drive the outer cladding (3) in rotation.

7. The casing (1) according to claims 5 and 6 wherein the internal connection part (51) is free in rotation in the external connection part (50), so that the rotation of the outer cladding (3) ensures the rotation of the inner cladding (4) by friction between the two claddings.

8. The casing (1) according to any of the preceding claims further comprising a third cladding in flexible material extending inside the inner cladding (4) to allow the injection or extraction of gas at the distal ends (31, 41) of the two claddings, respectively outer (3) and inner (4).

9. The casing (1) according to any of the preceding claims in combination with claim 2, further comprising:

 - removable clamping or ligature means (13) designed to clamp or ligature the two claddings (3, 4) over an intermediate zone (16) located between the respective proximal (30, 40) and distal (31, 41) ends of the two claddings;
 - at least one intermediate communication orifice (14) between the inside of the inner cladding (4) and the inter-cladding space (10), the said intermediate communication orifice (14) being provided on the inner cladding (4) between its proximal end (30) and said intermediate clamping or ligature zone (16); and
 - means (15) for closing the said intermediate communication orifice (14), in particular of trap type, the said closing means (15) being mobile between an open position and a closed position.

10. The casing (1) according to any of the preceding claims further comprising a longitudinal shadow strip partly covering the outer cladding (3) and made iin materal that is opaque or having strong light absorbance, the shadow strip particularly being added and positioned temporarily on the outer cladding (3) or secured onto the outer cladding (3).

11. A photosynthetic reactor (2) adapted for the culture of photosynthetic microorganisms, algae in particular, comprising :

 - at least one reaction casing (1) conforming to any of the preceding claims;

 - at least one return pipe (7) ensuring fluid connection between the first (11) and second (12) openings of the said reaction casing (1);
 - at least one circulation means (8) arranged in the said return pipe (7) and designed to set the liquid culture medium in circulation in the return pipe (7) and in the reaction casing (1);
 - at least one liquid injection means (9) arranged in the said return pipe (7) and designed to allow the injection of liquid into the reaction casing (1);
 - at least one gas injection means (61) arranged in the said return pipe (7) and designed to allow the injection of gas into the reaction casing (1); and
 - at least one gas escape means (60) arranged in the said return pipe (7) and designed to allow the escape of gas injected into the reaction casing (1).

12. A method for the culture of photosynthetic microorganisms, algae in particular, using a reactor conforming to claim 11 and comprising the following steps:

 - injecting a liquid culture medium into the reaction casing (1) at a controlled flow rate using the liquid injection means (9);
 - injecting a gas into the reaction casing (1) at a controlled flow rate using the gas injection means (61);
 - setting the liquid culture medium in circulation with the circulation means (8) ;
 - controlling the circulation means (8) and gas injection means (61) to set up in the reaction casing (1) a biphasic flow pattern of the gas/liquid culture medium of stratified flow, or slug or elongated bubble flow type.

13. The method according to claim 12 wherein the control step comprises a step to control the rate of circulation of the liquid in the reaction casing (1) at between about 0.1 and 1.0 m/s.

14. The method according to claims 12 or 13 wherein the circulation means (8) comprise a propeller (80) driven in rotation by a motor (81), and wherein the speed of rotation of the propeller is less than about 1000 rpm, and preferably less than about 100 rpm.

15. A method for manufacturing a reaction casing (1) conforming to any of claims 1 to 10 comprising the following steps:

 - fabricating the inner cladding (4), in particular by extruding a plastic material and blowing the extruded plastic;
 - fabricating an outer sheet (37) in plastic material in particular by calendering;

- wrapping the inner cladding (4) with the outer sheet (37) as far as the junction of two opposite edges; and
- welding the outer sheet (37) along its two opposite edges joined at the wrapping stage, so as to form the outer cladding (3) surrounding the inner cladding (4).

Fig. 1

Fig. 2a

Fig. 2b

Fig. 3a

Fig. 3b

Fig. 4

EP 2 499 229 B1

Fig. 5

Fig. 6a

Fig. 6b

30

Fig. 7a

Fig. 7b

Fig. 7c

Fig. 7d

Fig. 8a

Fig. 8b

Fig. 9a

Fig. 9b

EP 2 499 229 B1

Fig. 10a

Fig. 10b

Fig. 11a

Fig. 11b

33

Fig. 12a

Fig. 12b

Fig. 13a

Fig. 13b

Fig. 14

Fig. 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- GB 2118572 A
- ES 2193860 A1
- GB 2331762 A
- ES 2150389 A1
- FR 2685344 A1
- FR 2875511 A3
- FR 2621323
- FR 2361060
- FR 2324224
- WO 2009051479 A2
- WO 2008134010 A2
- WO 2009090549 A2